# EUROPÄISCHE PATENTANMELDUNG

(11) **EP 1 683 799 A1**
(43) Veröffentlichungstag der Anmeldung: **26.07.2006**
(21) Anmeldenummer: 05000522.2
(22) Anmeldetag: 12.01.2005
(51) Int. Cl.: C07D 495/04, A61K 31/4365, A61P 37/00

(54) **3-Amino-6aryl(bzw.6-heteroaryl)-4-R²-thieno[2,3-b]pyridin-2-carbonsäureamide, diese enthaltende pharmazeutische Zubereitungen und deren Verwendung als Inhibitoren der TNFalpha-Freisetzung**

(71) Anmelder: IBFB Pharma GmbH, 04103 Leipzig (DE)
(72) Erfinder: Reichelt, Claudia., 04299 Leipzig (DE); Ludwig, Alexander., 04275 Leipzig (DE); Leistner, Siegfried., 04349 Leipzig (DE)
(74) Vertreter: Schüssler, Andrea

(57) **Zusammenfassung**

Die Erfindung betrifft neue 3-Amino-6-aryl(heteroaryl)-4-R²-thieno[2,3-b]pyridin-2-carbonsäureamide der allgemeinen Formel 1,

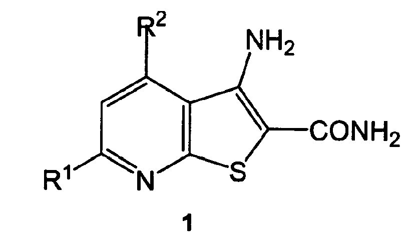

Verfahren zu deren Herstellung, pharmazeutische Zubereitungen, die diese Verbindungen und/oder deren Tautomere und gegebenenfalls daraus herstellbare physiologisch verträgliche Salze und/oder deren Solvate enthalten, sowie die Verwendung dieser Verbindungen, deren Tautomere, Salze oder Solvate als Inhibitoren der TNFα-Freisetzung.

## Beschreibung

### Technisches Gebiet

Die Erfindung betrifft neue 3-Amino-6-aryl(bzw. 6-heteroaryl)-4-R²-thieno[2,3-b]pyridin-2-carbonsäureamide der allgemeinen Formel 1, 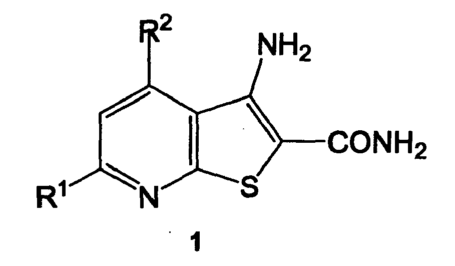 Verfahren zu deren Herstellung, pharmazeutische Zubereitungen, die diese Verbindungen und/oder deren Tautomere und gegebenenfalls daraus herstellbare physiologisch verträgliche Salze und/oder deren Solvate enthalten, sowie die Verwendung dieser Verbindungen, deren Tautomere, Salze oder Solvate als Inhibitoren der TNFα-Freisetzung.

### Stand der Technik

Das Zytokin Tumour necrosis factor (TNFα) ist eines von heute 17 bekannten Mitgliedern einer strukturell sehr ähnlichen Proteinfamilie. Seinen Namen verdankt es der Fähigkeit, eine Nekrose von transplantierten Tumorzellen im Mausmodell zu triggern. Neben seiner Apoptose-induzierenden Wirkung wurde sehr schnell erkannt, dass TNFα auch ganz maßgeblich in die Regulation der Entzündungsantwort und der Immunantwort eingebunden ist. Eine Überproduktion von TNFα oder die Aktivierung der TNFα-vermittelten Signalkaskaden spielen in der Pathogenese einer Vielzahl von Erkrankungen, wie z.B. Sepsis, cerebrale Form der Malaria, neurodegenarativen Erkrankungen wie z.B. Mb. Alzheimer, Mb. Parkinson, bei Diabetes mellitus, COPD/Asthma, Tumorerkrankungen und hier insbesondere Tumoren des blutbildenden Systems wie z.B. Leukämien und Lymphome, virale Erkrankungen und hier insbesondere retrovirale Erkrankungen wie z.B. das erworbene Immundefizienz Syndrom (AIDS), Guillain-Barre Syndrom, Rhinitis allergica, allergische Konjunktivitis, systemische Sklerodermie, Graft versus host disease (GvHD), Systemischer Lupus Erythematodes (SLE), Osteoporosis, Toxisches Schocksyndrom, Akute Glomerulonephritis, akute und chronische Schmerzen, Arteriosklerose, Herzinfarkt, Schlaganfall, bei Sarkoidose, Multiple Sklerose, Rheumatoid Arthritis (RA), Osteoarthritis, Colitis ulcerosa, Vasculitis, Uveitis, Mb. Crohn, Mb. Behcet, Myastenia Gravis und chronisch entzündlichen Hauterkrankungen wie Psoriasis, atopische Dermatitis, Ekzeme und Alopecie, eine zentrale Rolle (*Chen G, Goeddel DV (2002 TNF-R1 signaling: a beautiful pathway. Science 296 1634-1635; Ware CF (2003) The TNF superfamily. Cytokine & Growth Factor reviews 14 181-184; Dempsey PW (2003) The signaling adaptors and pathways activated by TNF superfamily. Cytokine & Growth Factor reviews 14 193-209).*

Bei TNFα handelt es sich um eines der wichtigsten pro-inflammatorischen Zytokine, der in die Pathogenese fast aller chronisch entzündlichen Erkrankungen maßgeblich eingebunden ist. TNFα, welches auch als Chachektin, Makrophagen-Cytotoxin (MCT), E tumor necrosis factor-a und als macrophage cytotoxic factor (MCF) beschrieben wurde, wird von verschiedensten Zellen nach Stimulation mit Lipopolysaccharid (LPS), Interferronen (IFN's), IL-2, Bradykinin, GM-CSF, Antigen-Antikörper-Komplexen, Substanz P und zahlreichen weiteren biologisch aktiven Verbindungen synthetisiert und sezerniert. TNFα wird unter physiologischen Bedingungen hauptsächlich von Makrophagen, T-Lymphozyten, Mikrogliazellen und NL-Zellen gebildet. Stimulierte und somit aktivierte Fibroblasten, glatte Muskelzellen, Astrozyten, Keratinozyten, Endothelzellen und Lungen-Epithelzellen sezernieren gleichfalls TNFα.

Humanes TNFα ist ein 17 kDa großes Protein, welches aus 157 Aminosäuren besteht und zu Dimeren und Trimeren assoziiert. Es existiert eine weitere höhermolekulare Variante dieses Moleküls mit einer Molmasse von 26 kDa, das als Transmembranprotein in der Zellmembran verankert ist. Man geht heute davon aus, dass zunächst die höhermolekulare Transmembranform synthetisiert wird, deren extrazelluläre Domäne durch das TNFα converting enzyme (TACE) abgespalten wird. Das lösliche TNFα zirkuliert als ein Homotrimer und bindet sich an seine spezifischen Rezeptoren an Zelloberflächen. Die Bindung von TNFα an seine Rezeptoren (TNFR1, TNFR2) bewirkt bei diesen eine konformative Änderung und Dimerisierung bzw. Clusterung der Rezeptoren, welche über eine Signalkaskade den biologischen Effekt von TNFα vermitteln. In zahlreichen Untersuchungen konnte gezeigt werden, dass über die Bindung von TNFα an den TNFR1 die meisten biologischen Effekte realisiert werden. Dies beinhaltet die Induktion der Apoptose über eine Aktivierung der Caspase 8 und nachfolgender Aktivierung der Caspasen 3,6 und 7, die dann unmittelbar zur Apoptose der Zelle führen.

Ein weiterer wichtiger Signalweg durch TNFα ist die Aktivierung von zwei wichtigen Transkriptionsfaktoren, dem nuclear factor-kappaB (NF-_{K}B) und c-Jun. Diese beiden Transkriptionsfaktoren spielen eine außerordentlich wichtige Rolle in der Regulation der Genexpression bei der Zelldifferenzierung, dem Zellwachstum, bei der Immun- und Entzündungsantwort, bei Zellstressregulationsvorgängen und bei der Tumorgenese. NF-_{K}B reguliert unter anderem die Gene für IL-1α, IL-1β, IL-2, IL-3, IL-6, IL-8, IL-12, TNFα, LT-α, IFN-α/β, G-CSF, M-CSF, GM-CSF, für den Zytokinrezeptor IL-2Rα, für die Adhäsionsmoleküle ICAM-1, VCAM-1, MAdCAM, E-Selektin, für die immunregulatorischen Moleküle leichte Kette des Igγ, MHC Class I und II, TCRα und β, β₂ Mikroglobulin, TAP1, iNOS und für die Akute Phase Proteine SAA, α₁-saures Glycoprotein und TSG-14/PTX3.

Über die tatsächliche physiologische Bedeutung der Bindung von TNFα an den TNFR2 existieren heute noch erhebliche Widersprüche. Deshalb ist die Aufdeckung der genauen molekularen Signaltransduktionsabläufe noch Gegenstand der Grundlagenforschung. Mehrheitlich geht man heute davon aus, dass die Bindung von TNFα an den TNFR2 auch die Mitogen-aktivierten Proteinkinase Kinasen (MAPKK) aktiviert, im speziellen die MEKK1 und die ASK1, die über eine Aktivierungskaskade zur Aktivierung der c-Jun Kinase (JNK) und damit zu einer Aktivierung des Transkriptionsfaktors c-Jun führt. In diesen Regulationsweg ist auch die Aktivierung der p38 Kinase eingebunden, die zur Aktivierung von p38 führt. Die Aktivierung von p38 ist essentiell für die Produktion der pro-inflammatorischen Zytokine IL-1β, TNFα und IL-6 und ist darüber hinaus auch verantwortlich für die Induktion und Expression der mit chronischen Entzündung vergesellschafteten Enzyme COX-2 und iNOS (*Ono K, Han J (2000) The p38 signal transduction pathway: activation and function. Cell Signal 12 1*-*13*). Über weitere Aktivierungswege werden auch die wichtigen Transkriptionsfaktoren activatingtranscription factor 2 (ATF2) und das Aktivatorprotein-1 (AP-1) induziert, welche unmittelbar stimulierenden Einfluss auf die Expression pro-inflammatorischer Moleküle wie E-Selectin, RANTES, IL-12, IL-6 und IL-8 ausüben (*Guicciardi ME, Gores GJ (2003)J Clin Invest 111 1813-1815*).

Die biologische Bedeutung von TNF erkannten erstmals 1969 GRANGER et al. (*Granger GA, Shacks SJ, Williams TW, Kolb WP (1969) Lymphocyte in vitro cytotoxicity: specific release of lymphotoxin-like materials from tuberculin-sensitive lymphoid cells. Nature 221 1155-1157*) die zeigen konnten, dass ein von Lymphozyten und Makrophagen sezemiertes Protein (Lymphotoxin) zur Lyse von Zellen, insbesondere von Tumorzellen, führt. 1984 konnten GRAY et al. (*Gray PW, Aggarwal BB, Benton CV, Briingman TS, Henzel WJ, Jarrett JA, Leung DW, Maffatt B, Ng P, Svedersky LP et al. (1984) Cloning and expression of cDNA for human lymphotoxin, a lymphokine with tumor necrosis activity. Nature 312 721-724)* und PENNICA et al. (*Pennica D, Nedwin GE, Hayflick JS, Seeburg PH, Deyrynck R, Palladino MA, Kohr WJ, Aggarwal BB, Goeddel DV (1984) Human tumor necrosis factor: precursor structure, expression and homology to lymphotoxin. Natur 312 724-729*) die cDNA für TNFα klonieren und das Protein exprimieren.

Die biologische Aktivität von TNFα wird hauptsächlich über zwei spezifische Rezeptortypen (TNFR1, TNFR2) vermittelt, die sich transmembran und mit einem extra- und intrazellulären Anteil auf einer Vielzahl Zellen des menschlichen Körpers befinden.

TNFα besitzt ein sehr breites Spektrum an biologischen Aktivitäten und reguliert fast alle Zellen. Er ist aus heutiger Sicht ein wesentlicher Mediator bei Entzündungs- und Immunreaktionen, aber auch bei der Apoptose, der Zelldifferenzierung, bei der Induktion von Fieber und zahlreichen weiteren pathophysiologischen Regulationsprozessen.

Eine zentrale Stellung nimmt TNFα bei der Endothelzellaktivierung während des Entzündungsprozesses ein. Hierbei stellt die Aktivierung der vaskulären Endothelzellen einen wesentlichen Schritt in der Initiationsphase der entzündlichen Reaktionen im Gewebe dar. So führen pro-inflammatorische Zytokine, mit TNFα an der Spitze, zur Expression endothelialer Adhäsionsmoleküle und chemotaktisch wirksamer Chemokine, die ihrerseits Makrophagen und T-Lymphozyten die Möglichkeit geben, am Endothel anzudocken und über eine aktive Wanderung ins entzündliche Gewebe (Extravasion) zu kommen. Man unterscheidet heute in diesem Zusammenhang eine lokale Wirkung von TNFα von einer systemischen. Die lokalen Effekte sind wie oben angeführt eine verstärkte Diapetese von Immun- und Entzündungszellen ins entzündliche Gewebe und eine starke Adhäsion von Thrombozyten an den Blutgefäßwänden. Der systemische Effekt von TNFα führt zu Ödemen, einer Verringerung des Blutvolumens, Hypoproteinämie, verbreitete intravaskuläre Blutgerinnung und in ihrer Maximalvariante zu multiplem Organversagen (septischer Schock).

TNFα bewirkt also eine lokale Aktivierung des vaskulären Endothels, eine Freisetzung von Stickoxid (NO) mit nachfolgender Steigerung der vaskulären Permeabilität, eine erhöhte Expression von Adhäsionsmolekülen und eine erhöhte Expression von "class II major histocompatibility molecules" (MHC II). Das Ergebnis ist ein Einwandern von Entzündungs- und Immunzellen, Antikörpern und Komplementfaktoren in das entzündliche Gewebe. TNFα verursacht gleichfalls in den lokalen Lymphknoten eine antigenspezifische Aktivierung der B- und T-Lymphozyten. Des Weiteren aktiviert TNFα Thrombozyten und verstärkt deren Adhäsion an den Gefäßwänden.

TNFα selbst induziert die Synthese anderer pro-inflammatorischer Zytokine wie IL-1, IL-6, IL-8 und GM-CSF und führt dadurch zu einem Circulus vitiosus des entzündlichen Prozesses. Zusätzlich ist TNFα noch maßgeblich in weitere pathophysiologische Prozesse, wie die Gelenkknorpelzerstörung bei rheumatischen Erkrankungen, Knochenresorptionsprozesse, Hemmung der Knochenbildung, Hemmung der Proteoglycansynthese und Induktion von Matrix Metalloproteinasen (MMP's) und Prostaglandin E₂ *(Mease P (2002) Psoriatic arthritis: The role of TNF inhibition and the effect of its inhibition with etanercept. Clin Exp Rheumatol 20 (Suppl. 28) S116-S121*) involviert.

**Übersicht der abgesicherten biologischen Wirkung von TNFα auf humane Zellen/Organe**

| **Zellart/Organe** | **Biologische Wirkung** |
|---|---|
| | |
| Endothelzelle | Zytokin-Freisetzung, Expression von Adhäsionsmolekülen, Freisetzung von Gerinnungsfaktoren, Induktion der induzierbaren Stickoxid Synthase (iNOS) |
| B-Lymphozyt | Verstärkung der Antikörperproduktion |
| T-Lymphozyt | T-Zell Aktivierung, Apoptose, Freisetzung von IFNγ, IL-2 |
| Gehirn | Induktion von Fieber und Schlaf |
| Leber | Freisetzung von "Akute Phase" Proteinen |
| Adipozyt | Hemmung der Lipoprotein-Lipase, Vermittlung der Insulin Resistenz |
| Fibroblast | Zellteilung, Induktion von MMP's und Zytokinen |
| Myozyt | Induktion der Apoptose, Funktionsstörungen |
| Osteoclast | Knochenresorption |
| Macrophage | Freisetzung von TNFα nach Stimulation durch Bakterien, Viren und Zytokine |
| Monozyt | Monozyten Aktivierung, Freisetzung von TNFα, IL-1, IL-6, iNOS |
| Thrombozyten | verstärkte Thrombozytenaggregation |

### Entwicklung von TNFα Inhibitoren

In der Vergangenheit gab es zahlreiche therapeutische Strategien, um die biologische Aktivität von TNFα zu hemmen und damit den chronischen Entzündungsprozess zu unterbrechen.
- An erster Stelle standen Bemühungen, die Synthese von TNFα zu hemmen. Hierzu kamen anti-inflammatorische Zytokine, wie z.B. das IL-10, Pentoxifylline, Thalidomid bzw.-Analoga, Kortikosteroide, Cyclosporin A, PDE-4 Inhibitoren und Antisense Oligonukleotide zum Einsatz.
   Am erfolgreichsten werden seit Jahren Kortikosteroide in der akuten Phase eines schweren entzündlichen Prozesses eingesetzt. Ihre breitere und besonders längere Anwendung ist auf Grund der schweren unerwünschten Arzneimittelwirkungen stark limitiert. Diese Gründe treffen auch für das gleichfalls seit Jahren zugelassene Immunsuppresivum Cyclosporin A zu. Pentoxifylline und Thalidomidanaloga zeigten in den klinischen Studien nur eine unzureichende therapeutische Wirksamkeit.
   Der Einsatz von PDE-4 Inhibitoren zeigt über die intrazelluläre Steigerung der cAMP Konzentration einen inhibierenden Einfluss auf die TNFα Freisetzung. Momentan sind mit Cilomilast, AWD 12-81 (GSK) und Roflumilast (Altana) drei Entwicklungskandidaten in der fortgeschrittenen klinischen Prüfung bzw. stehen kurz vor der Zulassung. Die klinische Anwendung dieser Substanzen geht jedoch auch mit unerwünschten Arzneimittelwirkungen, hauptsächlich emetischer Natur, einher. Die Antisense-Therapie befindet sich noch in einer sehr frühen Entwicklungsphase und hat zumindest in den ersten Tieruntersuchungen noch nicht die erhoffte Wirksamkeit nachweisen können.
- Ein weiterer Ansatz bestand in der Inhibierung des TNFα Prozessings durch Inhibitoren der Metalloproteinase TNF converting enzyme (TACE).
   Die Entwicklung von niedermolekularen TACE Inhibitoren befindet sich noch in der Phase der angewandten Grundlagenforschung. So beschrieben TSUKIDA et al. 2004 Hydroxamsäurederivate, die TACE in vitro hemmen (*Tsukida T, Moriyama H, Inoue Y, Kondo H, Yoshino K, Nishimura S (2004) Synthesis and biological activity of selective azasugar-based TACE inhibitors. Bioorg Med Chem Lett. 22 1569-1572*). Einige Matrix Metalloproteinase Inhibitoren hemmen auch unspezifisch TACE, sind aber auf Grund ihrer MMPinhibitorischen Aktivität für eine pharmazeutische Entwicklung nicht geeignet.
   WILLIAMS et al. konnten überraschend zeigen, dass der MMP Inhibitor BB-2275 paradoxerweise sowohl TACE, als auch das Shedding der TNFα Rezeptoren (TNFR1, TNFR2) hemmt und dadurch keinen TNFα inhibierenden Effekt hatte (*Williams LM, Gibbons DL, Gearing A, et al. (1996) Paradoxical effects of a synthetic metalloproteinase inhibitor that blocks both p55 and p75 TNF receptor shedding and TNF alpha processing in RA synovial membrane cell cultures. J Clin Invest* 97 *2833-2841*). Alle heute bekannten TACE Inhibitoren sind in ihrer hemmenden Wirkung unspezifisch, d.h. auch andere wichtige Metalloenzyme werden durch sie gehemmt, mit der Wahrscheinlichkeit unerwünschter (Neben)Wirkungen.
- In neuerer Zeit haben sich Strategien zur Blockierung des TNFα durch Antikörper gegen TNFα und lösliche TNF-Rezeptoren durchgesetzt.
   Zum jetzigen Zeitpunkt sind mit Remicade® und Humira™ zwei monoklonale anti-TNF Antikörper von der FDA und auch von der EMEA als anti-inflammatorische Therapeutika zugelassen worden.
   Remicade (Essex/Centocor) wurde durch die FDA 1998 für die Indikation Mb. Crohn und in 2000 für die Indikation Rheumatoide Arthritis zugelassen. Momentan laufen klinische Studien für die Anwendung bei Psoriasis vulgaris und Psoriasis arthropatica. Bei Remicade handelt es sich um einen chimaeren monoklonalen Antikörper gegen das humane TNFα. In den klinischen Studien zeigte das Präparat gute bis sehr gute Wirkeigenschaften beim Mb. Crohn. Jedoch wurde über teilweise erhebliche Nebenwirkungen, wie erhöhte Infektionsgefahr, Magen-Darm Beschwerden, Kopfschmerz und allergische Reaktionen berichtet. Ein Teil der Nebenwirkungen wird auf den Maus-Anteil des monoklonalen Antikörpers zurückgeführt, der vom menschlichen Organismus als "fremd" erkannt wird, wodurch Antikörper dagegen gebildet werden. Remicade wird intravenös verabreicht und die jährlichen Medikamentenkosten belaufen sich auf über $ 12.000 pro Patient.
   Humira (Abbott) ist für die Behandlung der Rheumatoiden Arthritis seit 2002 in USA und seit 2003 in Europa zugelassen. Klinische Studien zur Behandlung der Psoriasis vulgaris zeigten sehr gute Therapieerfolge. Als häufige Nebenwirkungen wurden Kopfschmerz, erhöhte Infektanfälligkeit, Magen-Darm Beschwerden und allergische Reaktionen beobachtet. Bei Humira handelt es sich um einen vollhumanisierten monoklonalen Antikörper gegen humanes TNFα. Das Präparat wird subcutan (s.c.) verabreicht. Die jährlichen Behandlungskosten belaufen sich auch bei diesem Präparat auf über $ 12.000 pro Patient.
   Enbrel (Immunex/Wyeth) wurde erstmals durch die FDA 1998 für die Indikation Rheumatoide Arthritis zugelassen und seit 2000 ist das Präparat auch auf dem europäischen Markt. Die Zulassung für die Indikation Psoriasis vulgaris und Psoriasis arthropatica wird noch 2004 von der FDA erwartet. Bei Enbrel handelt es sich um ein rekombinantes (CHO-Zellen) dimeres Fusionsprotein, bei dem zwei extrazelluläre Bindungsdomänen des p75-Anteils des TNF Rezeptors an den Fc-Anteil des humanen IgG1-Moleküls angekoppelt sind und dadurch lösliches TNFα im Blut/Gewebe abbinden und somit neutralisieren kann. Laut Herstellerangaben besitzt dieses Fusionsprotein ein geringes immunogenes Potential, wohingegen aber auch Fälle beschrieben wurden, in denen eine Antikörperbildung gegen das Fusionsprotein beobachtet wurde. Als häufige Nebenwirkungen wurden allergische Reaktionen, Infektanfälligkeit und die Bildung von Auto-Antikörpem (ANA) beschrieben. Das Präparat wird subcutan verabreicht und die jährlichen Behandlungskosten belaufen sich gleichfalls auf über $ 10.000 pro Patient.

Zusammenfassend kann festgestellt werden, dass sich das therapeutische Konzept der Hemmung von TNFα als biologischer Endpunkt, als tragfähig in der Klinik erwiesen hat. Momentan stehen nur proteinogene Präparate (monoklonale Antikörper, Fusionsproteine) zur Verfügung, die eine Reihe von unerwünschten Arzneimittelwirkungen aufweisen. Darüber hinaus ist deren intravenöse bzw. subkutane Applikationsform für Patienten sehr belastend und geht mit einer entsprechend schlechten Compliance einher. Die sehr hohen Herstellungs- und somit auch Behandlungskosten limitieren ebenfalls deren Einsatz.

Es besteht deshalb der dringende Bedarf, neuartige wirkungsstarke Inhibitoren der TNFα-Freisetzung zu entwickeln und in die Klinik einzuführen. Es besteht speziell das dringende Bedürfnis nach niedermolekularen, nicht-proteinogenen Hemmern der Freisetzung von TNFα, die oral verabreichbar sind und im Vergleich mit bekannten antiinflammatorischen und/oder antipyretischen Wirkstoffen bei großer Wirksamkeit eine bessere Verträglichkeit aufweisen. Zurzeit existieren noch keine bekannten niedermolekularen Verbindungen, die selektiv die Synthese von TNFα hemmen, ohne mit anderen Stoffwechselwegen zu interagieren.

Es ist daher die Aufgabe der vorliegenden Erfindung, neue Verbindungen bereitzustellen, die die Freisetzung von TNFα signifikant hemmen.

Man hat dabei herausgefunden, dass sich 3-Amino-6-aryl(bzw. 6-heteroaryl)-4-R²⁻thieno[2,3-b]pyridin-2-carbonsäureamide der allgemeinen Formel 1 zur Lösung der Aufgabe eignen:

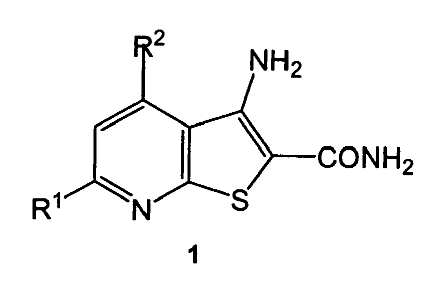

### Beschreibung der Erfindung

Die Erfindung betrifft 3-Amino-6-aryl(bzw. 6-heteroaryl)-4-R²-thieno[2,3-b]pyridin-2-carbonsäureamide der allgemeinen Formel 1, 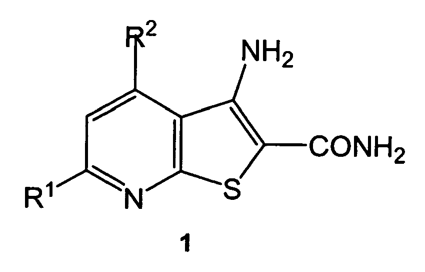 worin bedeuten:
R¹
   - Phenyl, 4-R³-Phenyl, 3-R³-Phenyl, 2-R³-Phenyl, 2-R³,3-R³-Phenyl, 2-R³,4-R³-Phenyl, 2-R³,5-R³-Phenyl, 2-R³,6-R³-Phenyl, 3-R³,4-R³-Phenyl, 3-R³,4-R³,5-R³-Phenyl,
   - 1-Naphthyl, 2-Naphthyl (jeweils ggf. mit R³ substituiert),
   - 1,2,3,4-Tetrahydronaphth-5-yl, 1,2,3,4-Tetrahydronaphth-6-yl (jeweils ggf. mit R³ substituiert),
   - 1-Anthryl, 2-Anthryl (jeweils ggf. mit R³ substituiert),
   - mono- oder bicyclische gesättigte oder ein- oder mehrfach ungesättigte Heterocyclen mit 5-14 Ringatomen, darunter 1-4 Heteroatome, die vorzugsweise N, O, S und Se sind (jeweils ggf. mit R³ substituiert),
R²
   - CH₂F, CHF₂, CF₃, CH₂Br, CHBr₂, CBr₃, CH₂Cl, CHCl₂, CCl₃, CHFCH₃, CF₂CH₃,
   - CH₂OAlk, CH₂SAlk, CH₂S(O)Alk, CH₂S(O)₂Alk ("Alk" jeweils C₁₋₃),
   - CH(OAlk)₂, CH(SAlk)₂ ("Alk" jeweils C₁₋₃);
R³
   - C₁₋₄Alkyl, C₁₋₄Alkoxy, Benzyl, Benzyloxy (jeweils ggf. mit R⁴ substituiert),
   - Trifluormethoxy, Difluormethoxy, Fluormethoxy,
   - C₃₋₇Cycloalkyl, C₃₋₇Cycloalkenyl, C₃₋₇Cycloalkoxy,
   - Alkylthio, Alkylsulfinyl und Alkylsulfonyl (jeweils C₁₋₄),
   - Phenyl, 4-R⁴-Phenyl, 3-R⁴-Phenyl, 2-R⁴-Phenyl, 2-R⁴,3-R⁴-Phenyl, 2-R⁴,4-R⁴-Phenyl, 2-R⁴,5-R⁴-Phenyl, 2-R⁴,6-R⁴-Phenyl, 3-R⁴,4-R⁴-Phenyl, 3-R⁴,4-R⁴,5-R⁴-Phenyl,
   - Phenoxy, 4-R⁴-Phenoxy, 3-R⁴-Phenoxy, 2-R⁴-Phenoxy, 2-R⁴,3-R⁴-Phenoxy, 2-R⁴,4-R⁴-Phenoxy, 2-R⁴,5-R⁴-Phenoxy, 2-R⁴,6-R⁴-Phenoxy, 3-R⁴,4-R⁴-Phenoxy, 3-R⁴,4-R⁴,5-R⁴-Phenoxy,
   - Pyrid-2-yl, Pyrid-3-yl, Pyrid-4-yl, Thien-2-yl, Fur-2-yl, Imidazol-2-yl, Oxazol-2-yl, Isoxazol-2-yl, Pyrrazol-4-yl,
   - C₁₋₇Alkacyl (ggf. mit R⁴ substituiert),
   - Formyl, Benzoyl, 1- und 2-Naphthoyl (jeweils ggf. mit R⁴ substituiert),
   - Fluor, Chlor, Brom, lod, Nitro, Hydroxy, Sulfhydryl, Cyano, Rhodano,
   - Carboxyl, C₁₋₄Alkoxycarbonyl;
   - CONH₂, CONHAlk und CONAlk₂ (mit "Alk" jeweils C₁₋₆),
   - SO₃H, SO₂OAlk, SO₂NHAlk, SO₂NAlk₂ (mit "Alk": C₁₋₅),
   - Chlor, Brom, Iod, Fluor,
   - Amino, C₁₋₆Alkylamino, Di(C₁₋₅)alkylamino,
   - Morpholino, Thiomorpholino, Thiomorpholino-S,S-Dioxid, Pyrrolidin-1-yl,
   - Piperidin-1-yl, Piperazin-1-yl, 4-Methyl-piperazin-1-yl, 4-Hydroxyethyl-1-piperazin-1-yl, 4-Phenyl-piperazin-1-yl,
   - Cycloalkylamino, C₃₋₁₄Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo-bzw. heterocyclischen Ringen mit R⁴ substituiert);
R³,4-R³
   - OCH₂O, OCH₂CH₂O;
R⁴
   - C₁₋₄Alkyl, C₁₋₄Alkoxy,
   - Trifluormethoxy, Difluormethoxy, Fluormethoxy,
   - Alkylthio, Alkylsulfinyl und Alkylsulfonyl (jeweils C₁₋₄),
   - Fluor, Chlor, Brom, lod, Nitro, Hydroxy, Sulfhydryl, Cyano, Rhodano,
   - Carboxyl, C₁₋₄Alkoxycarbonyl;
   - CONH₂, CONHAlk und CONAlk₂ (mit "Alk" jeweils C₁₋₆),
   - SO₃H, SO₂OAlk, SO₂NHAlk, SO₂NAlk₂ (mit "Alk": C₁₋₅),
   - Chlor, Brom, lod, Fluor,
   - Amino, C₁₋₆Alkylamino, Di(C₁₋₅)alkylamino,

### Ausgenommen vom Schutz ist die folgende Verbindung der allgemeinen Formel 1:

R¹: Thien-2-yl; R²: CF₃.

In der Vergangenheit sind bereits verschiedene primäre 3-Amino-thieno[2,3-b]pyridin-2-carbonsäureamide synthetisiert worden:

**Bekannte Verbindungen der allgemeinen Formel 1:**

| | | | |
|---|---|---|---|
| **R**^{**1**} | **R**^{**2**} | **R**^{**1**} | **R**^{**2**} |
| Me | Me | | |
| B.Tornetta, M.A.Siracusa, G.Rousisvalle und F.Guerrera: *Gazz.Chim.Ital.***108**, 57 (1978); | | | |

| | | | |
|---|---|---|---|
| Me | Ph | | |
| Ph | Me | | |
| V.P.Litvinov, Y.Sharanin et al.: *Izv.Akad. Nauk SSSR, Ser. Khim.* **1984**, 2760 | | | |

| **R**^{**1**} | **R**^{**2**} | **R**^{**1**} | **R**^{**2**} |
|---|---|---|---|
| 4-Cl-Ph | H | 4-Cl-Ph | 4-Cl-Ph |
| 4-Br-Ph | Me | 2-Furyl | Ph |
| Me | 4-Br-Ph | 2-Furyl | 4-F-Ph |
| Ph | 4-Cl-Ph | 2-Furyl | 4-Cl-Ph |
| Me | CONH₂ | 2-Furyl | 3-Pyridyl |
| Me | CONHC₄H₉(n) | 2-Furyl | 4-Br-Ph |
| Me | CONH(CH₂)₂OH | OH | Me |
| Me | COpiperidin-1-yl | 2-Naphthyl | 3-Pyridyl |
| G.Wagner, H.Vieweg, S.Leistner et al.: *Pharmazie* **45**, 102 (1990) | | | |

| | | | |
|---|---|---|---|
| Ph | Ph | Me | 3-Pyridyl |
| Me | 4-Pyridyl | | |
| G.Wagner, S.Leistner, H.Vieweg, U.Krasselt, J.Prantz: *Pharmazie* **48**, 514 (1993) | | | |

| | | | |
|---|---|---|---|
| Ph | 2-Thienyl | Me | 2-Furyl |
| Ph | 2-Furyl | Me | 4-Cl-Ph |
| F.A.Attaby: *Phosphorus, Sulfur, Silicon Relat.Elem*. **1998**, 139,1) | | | |

| | | | |
|---|---|---|---|
| 4-Cl-Ph | 2-Thienyl | | |
| Y.Sharanin et al.: *J.Org.Chem.USSR,(Engl.Transl.)* **1996**, 32, 1207 | | | |

| | | | |
|---|---|---|---|
| Me | 4-CH₃O-Ph | | |
| E.I.Kaigorodova et al.: *Chem.Herocycl.Compd.(Engl.Transl.)* **1996,** 32, 1234 | | | |

| | | | |
|---|---|---|---|
| Ph | 3-Pyridyl | | |
| A. Krauze: *Eur.J.Med.Chem.Chim.Ther*. **1999**, 34, 301 | | | |

| | | | |
|---|---|---|---|
| 2-Thienyl | CF₃ | | |
| M.I.Abdel-Monem et al.: *Pharmazie* **56**, 41 (2001) | | | |

Weitere knapp 200 Verbindungen der allgemeinen Formel 1 sind in zwei Patentanmeldungen (US 2004/0053957 A1 und US 2004/0180922 A1; Erfinder: Charles L. Cywin et alt.) der Firma Boehringer Ingelheim, Ridgefield, aufgeführt. 55 dieser Verbindungen weisen eine IKKβ-inhibitorische Wirkung auf (IC₅₀ < 10µM). Weitere 16 Verbindungen zeigen eine IKKα-inhibitorische Wirkung (IC₅₀ < 10µM). Diese Verbindungen weisen jedoch bezüglich R¹ und insbesondere bezüglich R² völlig andere Substituenten als die erfindungsgemäßen Verbindungen auf.

Erfindungsgemäße 3-Amino-6-aryl(bzw. 6-heteroaryl)thieno[2,3-b]pyridin-2-carbonsäureamide der allgemeinen Formel 1 sind als Inhibitoren der TNFα-Freisetzung bisher jedoch völlig unbekannt.

Die Begriffe "Alkyl, Alkenyl, Alkinyl, Alkoxy, usw.", auch in Wortzusammensetzungen wie Alkylsulfonyl, Alkylamino oder Alkoxycarbonyl usw. bedeuten sowohl die unverzweigten wie auch die möglichen verzweigten Verbindungen. Ebenso bedeuten "Alkenyl und Alkinyl" die entsprechend möglichen einfach oder mehrfach ungesättigten Verbindungen. Das gleiche gilt auch für die entsprechenden cyclischen Verbindungen.

Die Erfindung betrifft auch physiologisch verträgliche Salze der Verbindungen der allgemeinen Formel 1.

Die physiologisch verträglichen Salze werden auf übliche Weise durch Umsetzung basischer Verbindungen der allgemeinen Formeln 1 mit anorganischen oder organischen Säuren, ggf. auch bei Vorliegen von Verbindungen mit aciden Eigenschaften, wenn z.B. einer der Substituenten R¹ oder R² in diesen Verbindungen -COOH bzw. -SO₃H bedeutet, durch Neutralisation mit anorganischen oder organischen Basen, erhalten.

Als anorganische Säuren kommen vorzugsweise Salzsäure, Schwefelsäure, Salpetersäure oder Bromwasserstoffsäure, als organische Säuren zum Beispiel Ameisensäure, Essigsäure, Propionsäure, Glykolsäure, Milchsäure, Mandelsäure, Weinsäure, Äpfelsäure, Zitronensäure, Malonsäure, Maleinsäure, Fumarsäure, Succinsäure, Alginsäure, Benzoesäure, 2-, 3- und 4-Alkyloxy- und Acyloxybenzoesäuren, Ascorbinsäure, C₁-C₃,Alkylsulfonsäuren, Benzolsulfonsäure, Nicotinsäure, Isonicotinsäure und Aminosäuren zur Anwendung.

Als anorganische Basen kommen zum Beispiel Ammoniak, Natron- und Kalilauge sowie als organische Basen Alkylamine, C₁-C₃, Pyridin, Chinolin, Isochinolin, Piperazin und -Derivate, Picoline, Chinaldin oder Pyrimidin zur Anwendung.

Weiterhin können physiologisch verträgliche Salze der Verbindungen gemäß der allgemeinen Formeln 1 dadurch gewonnen werden, dass jene Substanzen, die als Substituenten eine tertiäre Amino-Gruppe besitzen, in prinzipiell bekannter Weise mit alkylierenden Agentien - wie zum Beispiel Alkyl- oder Aralkylhalogeniden - in die entsprechenden quartemären Ammoniumsalze übergeführt werden können.

Die Erfindung betrifft auch Solvate der Verbindungen, einschließlich der pharmazeutisch akzeptablen Salze, Säuren, Basen und Ester sowie deren aktive Metabolite und gegebenenfalls deren Tautomere gemäß der allgemeinen Formeln 1 einschließlich Prodrug-Formulierungen. Prodrug-Formulierungen umfassen hierbei alle jene Substanzen, die durch einfache Transformation einschließlich Hydrolyse, Oxidation, oder Reduktion entweder enzymatisch, metabolisch oder auf andere Art und Weise entstehen. Insbesondere, wenn ein solches Prodrug dieser erfindungsgemäßen Verbindungen einem Patienten appliziert wurde, und dieses Prodrug in eine Substanz der allgemeinen Formeln 1 transformiert wird, wodurch der gewünschte pharmakologische Effekt erzielt wird.

Die durch die erfindungsgemäßen Verbindungen behandelbaren Erkrankungen schließen alle ein, bei denen TNF-alpha eine Rolle spielt und die durch eine Hemmung oder Inhibierung desselben positiv zu beeinflussen sind, z.B. chronische Entzündungserkrankungen, Autoimmun-Erkrankungen, cardiovaskuläre Erkrankungen, virale Erkrankungen und hier insbesondere retrovirale Erkrankungen wie z.B. das erworbene Immundefizienz Syndrom (AIDS) sowie Krebs, insbesondere Entartungen des blutbildenden Systems. Insbesondere sind dies Rheumatoide Arthritis, Osteoarthritis, Osteoporosis, Asthma bronchiale, chronische obstruktive pulmonäre Erkrankung (COPD), Multiple Sklerose, Sepsis, cerebrale Form der Malaria, neurodegenerativen Erkrankungen wie z.B. Mb. Alzheimer, Mb. Parkinson, Guillain-Barre-Syndrom, Crohns Disease, Colitis ulcerosa, Psoriasis, Graft-versus-Host-Disease (GvHD), systemischer Lupus erythomatodes (SLE), Vasculitis, Uveitis, insulin-abhängiger Diabetes mellitus, Respiratorisches Distress-Syndrom beim Erwachsenen (ARDS), multiples Organversagen nach Trauma, aktute Glomerulonephritis, akute und chronische Schmerzen, Arteriosklerose, Herzinfarkt, Schlaganfall, entzündliche Dermatosen, atopische Dermatitis, Psoriasis vulgaris, Alopecie, Rhinitis allergica, allergische Konjunktivitis, akute Meningitis, Myastenia Gravis, Sklerodermie und Sarkoidose.

Die erfindungsgemäßen Substanzen haben sich als Inhibitoren der TNFα Freisetzung, z.B. vornehmlich aus aktivierten Makrophagen, aber auch aus Mastzellen, Fibroblasten, Basophilen, Granulozyten, Endothelzellen, aktivierten Lymphozyten und Astrozyten (im Gehirn) bewährt.

Die erfindungsgemäßen Verbindungen können auf verschieden Wegen verabreicht werden, z.B. oral, parenteral, kutan, subkutan, intravenös, intramuskulär, rektal oder inhalativ. Die Verbindung wird einem Patienten, der eine Therapie einer unter das Indikationsspektrum der erfindungsgemäßen Verbindungen fallenden Krankheit bedarf, über einen vom Arzt zu bestimmenden Zeitraum verabreicht. Die Verbindung kann sowohl Menschen als auch anderen Säugern verabreicht werden.

Die Dosierung der erfindungsgemäßen Verbindungen wird vom Arzt anhand der patientenspezifischen Parameter wie z.B. Alter, Gewicht, Geschlecht, Schwere der Erkrankung, etc. bestimmt. Bevorzugt beträgt die Dosierung zwischen 0,001 mg/kg bis 100.000 mg/kg Körpergewicht, bevorzugt 0,01 bis 10.000 mg/kg Körpergewicht und ganz bevorzugt 0,1 bis 1.000 mg/kg Körpergewicht.

Entsprechend der Art der Verabreichung wird das Medikament in geeigneter Weise formuliert, z.B. in Form von Lösungen bzw. Suspensionen, einfachen oder dragierten Tabletten, Hart- oder Weichgelatinekapseln, Pulver zur Rekonstitution vor Gebrauch, Aerosolen, Inhalationssprays, Wirkstoffpflastem, Granulaten, Suppositorien, Ovula, Injektionspräparaten, Cremes, Salben, Gels, Mikrospheren, Implantaten, die nach üblichen galenischen Verfahren hergestellt werden.

Die erfindungsgemäßen Verbindungen können gegebenenfalls zusammen mit weiteren Wirkstoffen und mit in pharmazeutischen Zusammensetzungen üblichen Exzipientien formuliert werden, z.B. je nach herzustellendem Präparat Talk, Gummi arabicum, Lactose, Stärke, Magnesiumstearat, Kakaobutter, wäßrige und nichtwäßrige Träger, Fettkörper mit tierischem oder pflanzlichem Ursprung, Paraffinderivate, Glykole (insbesondere Polytethylenglykol), verschiedene Weichmacher, Dispergiermittel oder Emulgatoren, pharmazeutisch verträgliche Gase (z.B. Luft, Sauerstoff, Kohlendioxid usw.), Konservierungsstoffe.

Zur Herstellung flüssiger Präparate können Additive wie Natriumchloridlösung, Ethanol, Sorbit, Glycerin, Olivenöl, Mandelöl, Propylenglycol oder Ethylenglycol verwendet werden.

Bei der Verwendung von Infusions- oder Injektionslösungen sind diese sind bevorzugt wäßrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Geringe Mengen an Antibiotika können auch zugesetzt werden, um die Beibehaltung der Sterilität zu gewährleisten.

Weiter bevorzugt werden Inhalationszusammensetzungen, z.B. in Form von Aerosolen, Sprays, oder als mikronisiertes Pulver hergestellt. Dazu werden die erfindungsgemäßen Verbindungen entweder als in pharmazeutisch üblichen Lösungsmitteln gelöst bzw. suspendiert und mittels Überdruck in einem bestimmten Volumen fein verteilt und inhaliert. Ein entsprechendes Vorgehen erfolgt bei den zu inhalierenden Festsubstanzen, die gleichfalls mittels Überdruck fein verteilt und inhaliert werden. Ebenfalls andere als mit Überdruck funktionierende Applikatoren sind hierbei eingeschlossen.

Die Erfindung betrifft auch pharmazeutische Zubereitungen, die eine therapeutisch wirksame Menge des aktiven Inhaltsstoffs (erfindungsgemäße Verbindung der Formel 1 zusammen mit organischen oder anorganischen festen oder flüssigen pharmazeutisch verträglichen Trägern, die für die beabsichtigte Verabreichung geeignet sind, und die mit den aktiven Inhaltsstoffen nicht nachteilig wechselwirken, enthalten.

Die Erfindung betrifft auch Verfahren zur Herstellung pharmazeutischer Zubereitungen, die dadurch gekennzeichnet sind, dass die erfindungsgemäße Verbindung mit einem pharmazeutisch verträglichen Träger vermischt wird.

Die erfindungsgemäßen Verbindungen eignen sich auch im Rahmen von Kombinationstherapien mit schon bekannten Wirkstoffen zur Behandlung der oben genannten Erkrankungen. Dabei sollen überraschende Synergieeffekte zur Steigerung der therapeutischen Wirksamkeit der erfindungsgemäßen Substanzen genutzt werden. Die Kombination kann zum einen darin bestehen, eine einzige pharmazeutische Zusammensetzung anzubieten, die mindestens eine der erfindungsgemäßen Verbindungen in Kombination mit einem oder mehrere der nachfolgend genannten Wirkstoffen enthält oder dem Patienten werden gleichzeitig oder zeitlich versetzt mehrere Mittel, die einen oder mehreren der nachfolgenden Wirkstoffe enthalten, verabreicht.

Es ist bevorzugt eine oder mehrere der erfindungsgemäßen Verbindungen mit einem oder mehreren der folgenden Wirkstoffe zu kombinieren:
- Corticosteroide
- (monoklonale) Antikörpern gegen TNF-alpha oder andere Wirkstoffe, die die Bildung bzw. Freisetzung von TNF-alpha oder die Aktivität von TNF-alpha hemmen (z.B. rekombinante TNFα-Rezeptorkonstrukte)
- Zytokin-Antagonisten (z.B. IL-1β, IL-6, IL-12)
- Chemokin-Antagonisten
- Zytokin-Agonisten (z.B. IL-10)
- immunmodulatorische Wirkstoffe wie z.B. Cyclosporin A, Methodrexat, Leflunomid, D-Penicillamin, Auranofine
- Substanz P-Antagonisten
- Bradykinin-Antagonisten
- PAF-Antagonisten
- Adenosin-Rezeptor-Antagonisten
- Antibiotika/Virostatika
- α-Mimetika
- Zytostatika
- β₂-Adrenoceptor Agonisten (z.B. Terbutalin, Salbutanol, Salmetanol, Fenoterol, Formoterol)
- Leukotrien-Antagonisten (entweder Enzym-Inhibitoren [wie 5-Lipoxygenaseinhibitoren oder Arachidonsäure-Enzyminhibitoren] oder Rezeptorantagonisten), z.B. Pranlukast, Montelukast, Zafirlukast, Zileuton
- Antihistaminika (bevorzugt solche mit Mastzellen-stabilisierenden Eigenschaften oder Leukotrien-antagonisierenden Aspekten, wie z.B. Loratadin, Astemizol, Mizolastin, , Olopatadin
- Theophyllin
- Muscarinrezeptor-Antagonisten, z.B. Spiriva

Die Kombination mit oben aufgeführten Arzneimitteln bzw. Wirkprinzipien dient besonders dazu, den akut zu behandelnden Krankheitszustand in einem möglichst frühen Stadium in seiner Manifestation zu beeinflussen und nicht chronisch werden zu lassen, da die erfindungsgemäßen Verbindungen in Kombination mit den anderen Wirkstoffen komplementäre/additive Aspekte ermöglichen. In der Kombination ergibt sich ein positiver Effekt u.a. daraus, dass eine geringere Substanzmenge pro Prinzip angewendet werden kann und damit zum einen eine Verbesserung des therapeutischen Effektes, geringere unerwünschte Arzneimittelwirkungen und zum anderen ein Spareffekt zu erreichen ist.

Abhängig von der Krankheitsausprägung und den zugrunde liegenden Symptomen können die erfindungsgemäßen Verbindungen zu den anderen Wirkstoffen in der Kombination im Verhältnis von 1:10.000 bis 10.000:1 vorliegen.

Die Erfindung betrifft weiterhin Verfahren zur Herstellung der erfindungsgemäßen Verbindungen.

Die erfindungsgemäßen Verfahren zur Herstellung der Verbindungen der allgemeinen Formeln 1 mit den zuvor aufgeführten Bedeutungen von R¹ und R² sind gekennzeichnet durch folgende Verfahrensweisen:
**A)** - Umsetzung der literaturbekannten bzw. nach prinzipiell bekannten Methoden dargestellten und bislang literaturunbekannten Pyridin-3-carbonitrile der allgemeinen Formel 2, 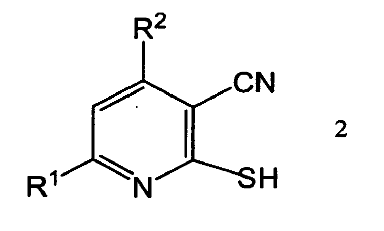 mit identischer Bedeutung von R¹ und R² wie oben,
   in an sich bekannter Weise mit 2-Chloracetamid, ClCH₂CONH₂, oder 2-Bromacetamid, BrCH₂CONH₂, in methanolischer oder ethanolischer Lösung in Gegenwart eines Natriumalkoxides, vorzugsweise Natriummethoxid oder Natriumethoxid, zunächst zu den analogen 3-Amino-thieno[2,3-b]pyridin-2-carbonsäureamiden der allgemeinen Formel 4, 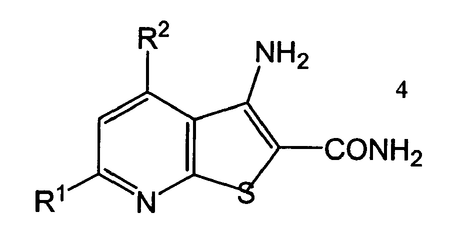 mit identischer Bedeutung von R¹ und R²;
**B)** - Die Verbindungen der allgemeinen Formel 4 (mit identischer Bedeutung von R¹ und R², wie oben) sind aus den Verbindungen der allgemeinen Formel 2, auch dadurch darstellbar, dass diese Verbindungen mit 2-Choracetamid zunächst in vorzugsweise ethanolischer Lösung in Gegenwart von vorzugsweise Triethylamin oder in wasserfreier acetonischer Lösung in Gegenwart von Natrium- oder Kaliumhydrogencarbonat zu den Verbindungen der allgemeinen Formel 3, 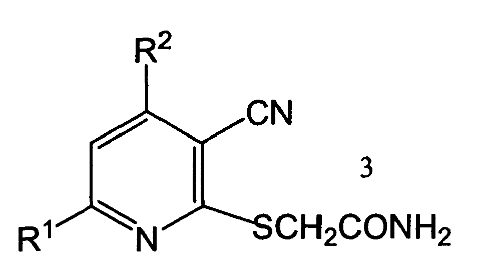 worin R¹ und R² die oben genannten Bedeutungen aufweisen, umgesetzt werden und diese Verbindungen in einem weiteren Syntheseschritt in vorzugsweise wasserfreier ethanolischer Lösung mit einer nur geringen katalytischen Menge Natriummethoxid oder Natriumethoxid durch Erhitzen unter Rückfluss gleichfalls in die oben genannten Verbindungen der allgemeinen Formel 4 übergeführt werden;

Entsprechend der vorliegenden Erfindung können die unter A) und B) beschriebenen Verfahren variiert werden.

Weitere Ausführungsformen der Erfindung können beispielsweise darin bestehen, dass die Darstellung der bicyclischen 3-Amino-6-aryl(heteroaryl)-4-R²-thieno[2,3-b]pyridin-2-carbonsäureamide Pyrimidin-2,4-dione der allgemeinen Formel 1 in weiten Grenzen variierbar ist.

### Methodenbeschreibung der Hemmung der TNFα Freisetzung nach LPS Stimulation von humanem Vollblut

Die Stimulierung isolierter Leukozyten für die Freisetzung von Zytokinen kann auf verschiedenen Wegen erfolgen. Lipopolysaccharide (LPS) stellen einen Stimulus für die Untersuchung der Freisetzung von TNFα dar. LPS ist Bestandteil bakterieller Zellwände und wird beim Abtöten der Bakterien (durch Antibiotika oder das natürliche Immunsystem) freigesetzt. LPS stimuliert insbesondere die Aktivität phagozytierender Leukozyten (Gewebsmakrophagen, Granulozyten, Monozyten) und verursacht die Infiltration von Leukozyten vom peripheren Blut in das betroffene Gewebe. Ein Zytokin von besonderer Bedeutung für diese Mechanismen ist TNFα, das in großen Mengen durch die betroffenen Zellen sezemiert wird. Hauptquelle dabei sind Monozyten und Makrophagen. TNFα initiiert und prolongiert den Entzündungsprozess im Zusammenspiel mit anderen Mediatoren.

Für die Untersuchung des Effektes auf die LPS-induzierte TNFα-Freisetzung wurde eine Methode verwendet, die von MARX et al. (*Marx D, Tassabehji M, Heer S, Hüttenbrink KB, Szelenyi I (2002) Pulmonary Pharmacology & Therapeutics 15 7-15*) beschrieben wurde. Die Methodenbeschreibung in verkürzter Darstellung:
Humanes Blut verschiedener Spendern wird durch Zusatz von 10 mM Na-Citrat ungerinnbar gemacht und 1:5 mit RPMI 1640 Zellkulturmedium verdünnt. Die Verbindungen der allgemeinen Formel 1 sowie die Kontrollsubstanzen (z.B. Dexamethason) wurden den Blutproben in verschiedenen Konzentrationen hinzugefügt. 15 Minuten später wurden die Leukozyten durch Zusatz von Lipopolysacchariden (LPS) (aus Salmonella abortus equi) in einer Endkonzentration von 1 µg/ml stimuliert. Nach Inkubation der Testansätze für 24 Stunden bei 37°C. und unter 5% CO₂ in wassergesättigter Luft, wurde das Blut zentrifugiert und die Konzentration an TNFα im zellfreien Überstand unter Verwendung eines käuflichen ELISA (BD Biosciences) nach Angaben des Herstellers exakt vermessen.

Für in der Erfindung beschriebene Verbindungen der allgemeinen Formel 1 wurden für die Hemmung der TNFα- Freisetzung EC₅₀-Werte im Bereich von < 1 nM bis < 100 nM (Positivkontrolle: Dexamethason®) bestimmt.

Die nachfolgende Auflistung beinhaltet Beispiele erfindungsgemäßer Substanzen der allgemeinen Formel 1, die die TNFα-Freisetzung mit einem EC₅₀ < 1 nM hemmen:
3-Amino-6-phenyl-4-trifluormethyl-thieno[2,3-b]pyridin-2-carbonsäureamid;
3-Amino-4-difluormethyl-6-(4-methyl-phenyl)-thieno[2,3-b]pyridin-2-carbonsäureamid;
3-Amino-6-(4-methyl-phenyl)-4-trifluormethyl-thieno[2,3-b]pyridin-2-carbonsäureamid;
3-Amino-6-(4-methoxy-phenyl)-4-trifluormethyl-thieno[2,3-b]pyridin -2-carbonsäureamid;
3-Amino-6-(4-ethyl-phenyl)-4-trifluormethyl-thieno[2,3-b]pyridin-2-carbonsäureamid;
3-Amino-6-(4-ethoxy-phenyl)-4-trifluormethyl-thieno[2,3-b]pyridin -2-carbonsäureamid;
3-Amino-6-(4-isopropyl-phenyl)-4-trifluormethyl-thieno[2,3-b]pyridin -2-carbonsäureamid;
3-Amino-6-(4-propyl-phenyl)-4-trifluormethyl-thieno[2,3-b]pyridin-2-carbonsäureamid;
3-Amino-6-(4-methylthio-phenyl)-4-trifluormethyl-thieno[2,3-b]pyridin-2-carbonsäureamid
3-Amino-6-(3,4-dioxomethylen-phenyl)-4-trifluormethyl-thieno[2,3-b]pyridin -2-carbonsäureamid;
3-Amino-6-(3,4-dioxoethylen-phenyl)-4-trifluormethyl-thieno[2,3-b]pyridin -2-carbonsäureamid;
3-Amino-6-(3,4-dimethyl-phenyl)-4-trifluormethyl-thieno[2,3-b]pyridin -2-carbonsäureamid;
3-Amino-6-(2-naphthyl)-4-trifluormethyl-thieno[2,3-b]pyridin-2-carbonsäureamid;
3-Amino-6-(2-thienyl)-4-trifluormethyl-thieno[2,3-b]pyridin-2-carbonsäureamid;
3-Amino-6-(4-chlor-phenyl)-4-trifluormethyl-thieno[2,3-b]pyridin-2-carbonsäureamid;
3-Amino-6-(4-brom-phenyl)-4-trifluormethyl-thieno[2,3-b]pyridin-2-carbonsäureamid;
3-Amino-6-(4-fluor-phenyl)-4-trifluormethyl-thieno[2,3-b]pyridin-2-carbonsäureamid;

Die nachfolgende Auflistung beinhaltet Beispiele erfindungsgemäßer Substanzen der allgemeinen Formel 1, die die Freisetzung von TNFα mit einem EC₅₀ >1 und <100 nM hemmen:
3-Amino-6-(4-*tert*.butyl-phenyl)-4-trifluormethyl-thieno[2,3-b]pyridin -2-carbonsäureamid;
3-Amino-6-(4-methylsulfinyl-phenyl)-4-trifluormethyl-thieno[2,3-b]pyridin -2-carbonsäureamid;
3-Amino-6-(2-furyl)-4-trifluormethyl-thieno[2,3-b]pyridin -2-carbonsäureamid;
3-Amino-4-methoxymethyl-6-(4-methyl-phenyl)thieno[2,3-b]pyridin -2-carbonsäureamid;

Die Erfindung wird durch die nachfolgenden Beispiele näher erläutert:

### 3-Amino-4-difluormethyl-6-(4-methyl-phenyl)thieno[2,3-b]pyridin-2-carbonsäureamid (Verbindung der allgemeinen Formel 1; Beispiel 1)

Zu einer Suspension von 1,38 g (5 mmol) 6-(4-Methyl-phenyl)-2-mercapto-4-difluor-methyl-nicotinonitril (Verbindung 2/1 der allgemeinen Formel 2) und 0,68 g (10 mmol) Natriumethanolat in 15 ml wasserfreiem Ethanol werden 0,56 g (6 mmol) 2-Chlor-acetamid eingetragen und der Ansatz zwei Std. unter Rückfluss erhitzt. Nach dem Abkühlen wurde der Niederschlag abgesaugt, mit wenig Ethanol und Wasser gewaschen und anschließend bei ca. 50 °C im Vakuum getrocknet.
Gelbes, mikrokristallines Pulver.
Ausbeute: 1.43 g (85,8%).
Schmb.: 221 - 223 °C (Ethanol).
C₁₆H₁₃F₂N₃OS (333,4). MS (ESI): 334.0 m/e [M+H]⁺.
HPLC: 99,8 %. t_{R}: 16,6 min.

### 3-Amino-6-(4-fluor-phenyl)-4-trifluormethyl-thieno[2,3-b]pyridin-2-carbonsäureamid (Verbindung der allgemeinen Formel 1, Beispiel 22)

Zu einer Suspension von 1,49 g (5 mmol) 6-(4-Fluor-phenyl)-2-mercapto-4-trifluormethyl-nicotinonitril (Verbindung 2/22 der allgemeinen Formel 2) und 0,68 g (10 mmol) Natriumethanolat in 25 ml wasserfreiem Ethanol werden 0,48 g (5 mmol) 2-Chlor-acetamid eingetragen und der Ansatz zwei Stunden unter Rückfluss erhitzt. Nach dem Abkühlen wurde die Reaktionsmischung in 100 ml Wasser eingetragen und i.Vak. eingeengt. Der Niederschlag wurde abgesaugt und bei ca. 50 °C im Vakuum getrocknet.
Ockerfarbene Kristalle.
Ausbeute: 1.31 g (74,0%).
Schmb.: 246 - 249 °C (Ethanol).
C₁₅H₉F₄N₃OS (355,3). MS (ESI): 356,0 m/e [M+H]⁺.
HPLC: 97,0 %. t_{R}: 3.98 min.

Analog dargestellte Verbindungen der allgemeinen Formel 1 sind als exemplarische Beispiele in der nachfolgenden Tabelle 1 aufgeführt:

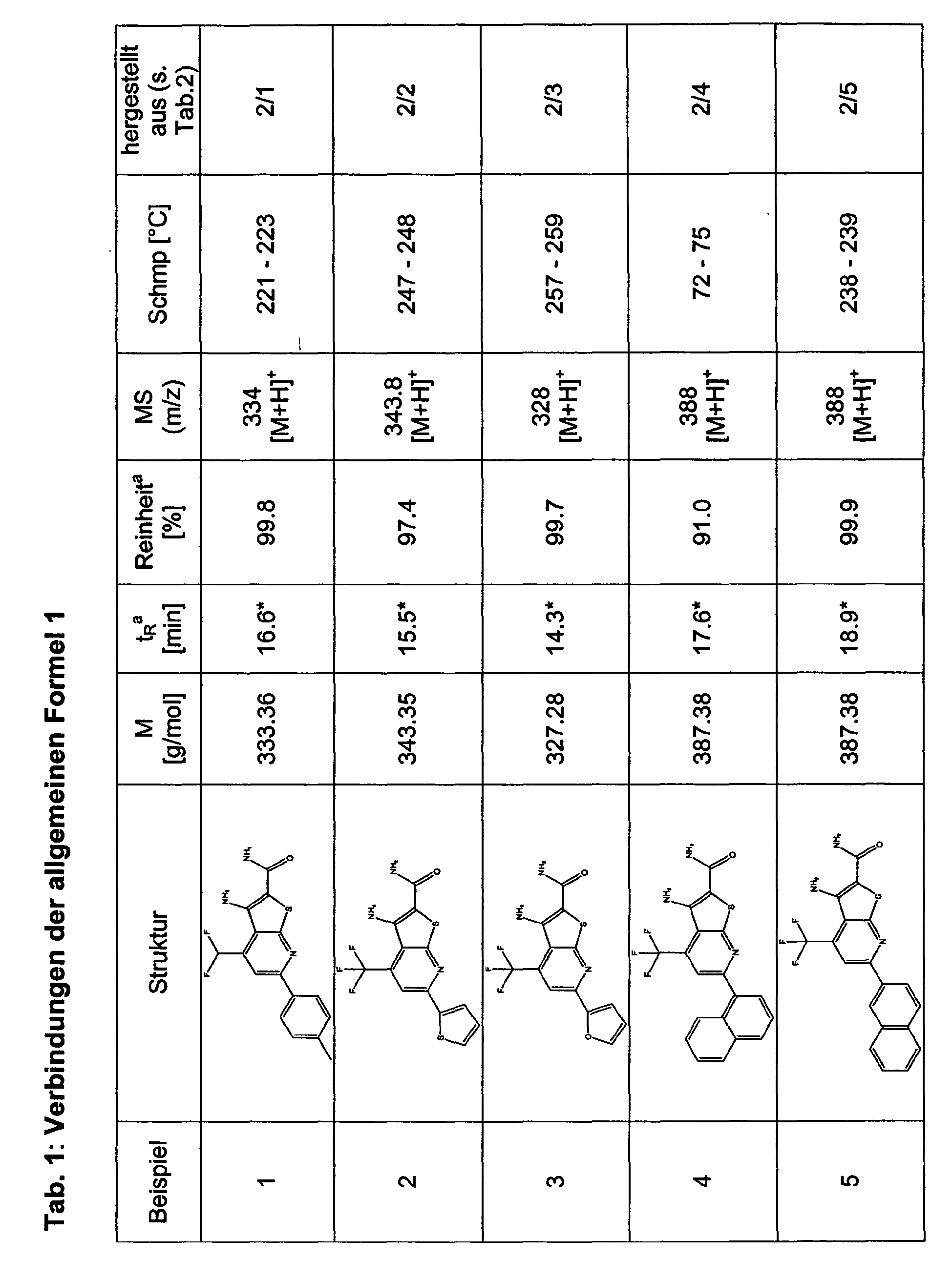

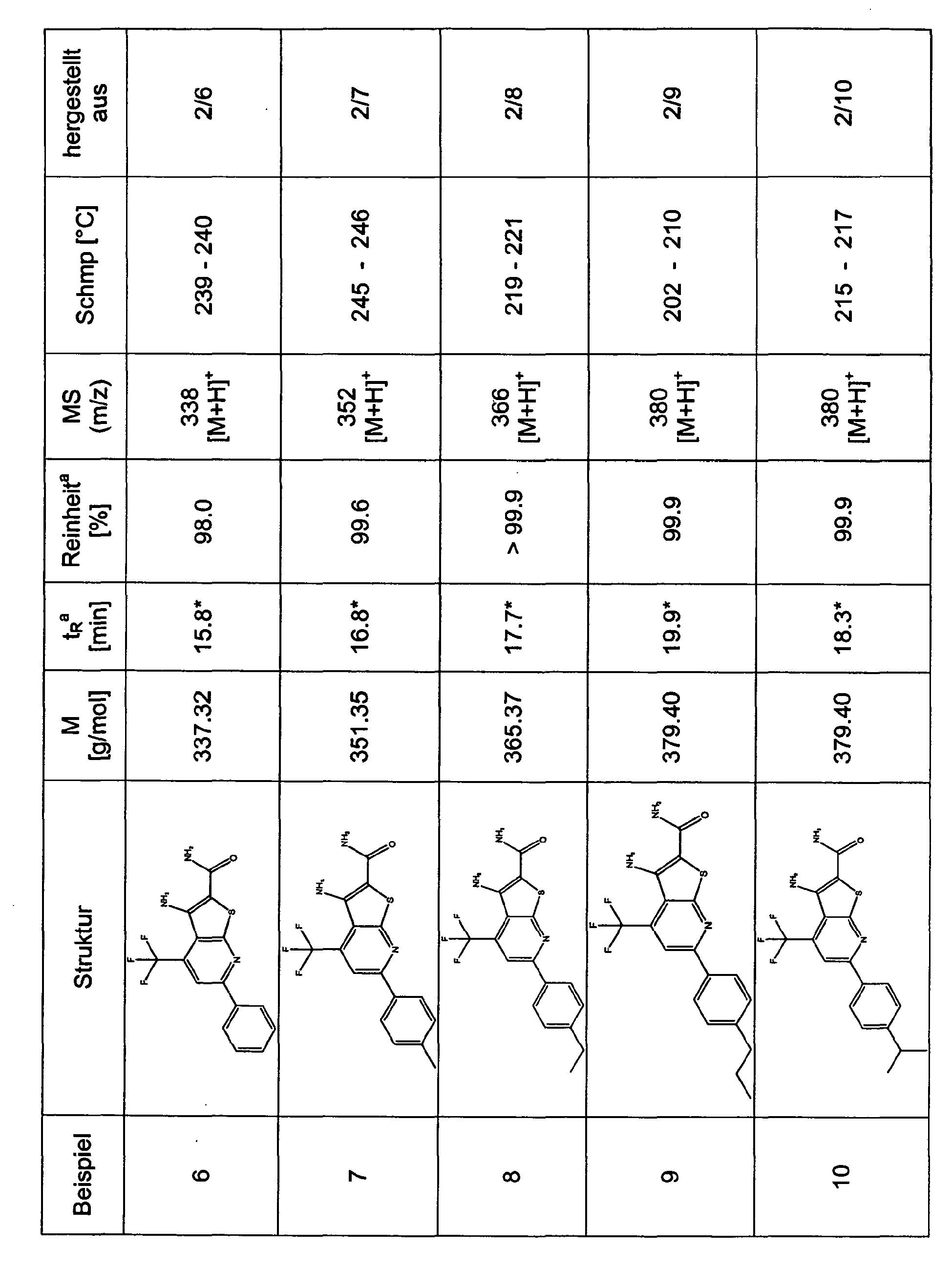

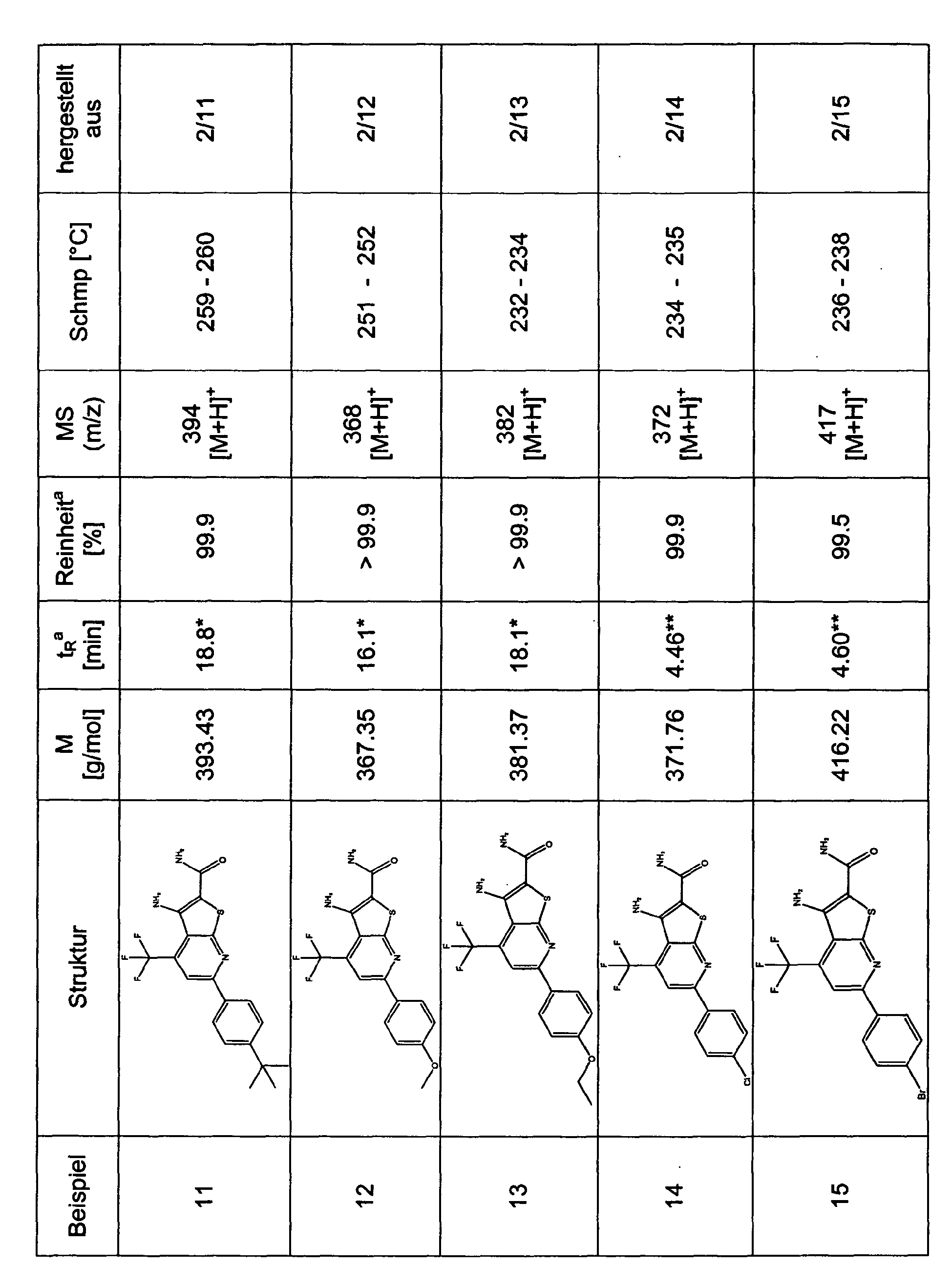

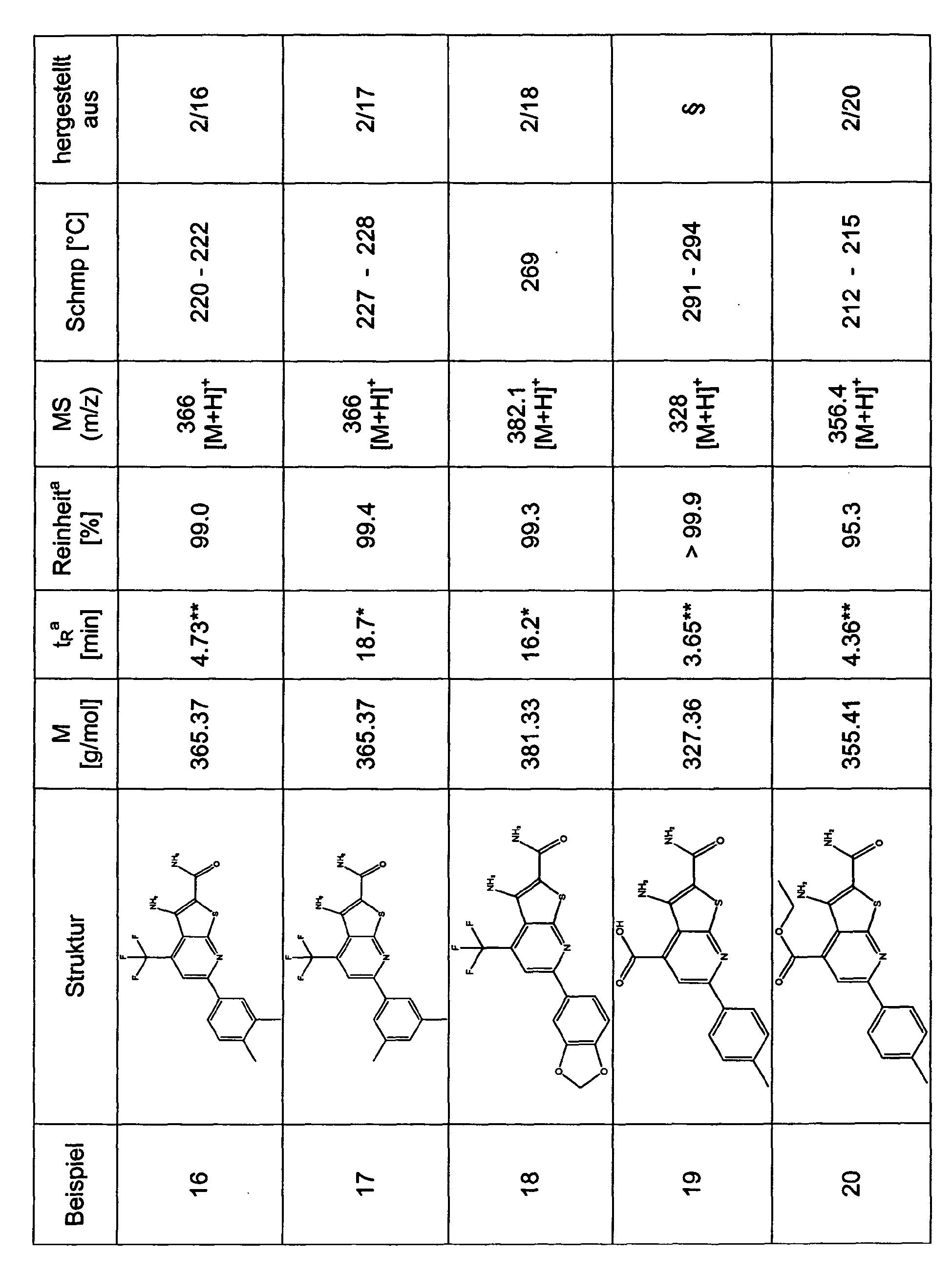

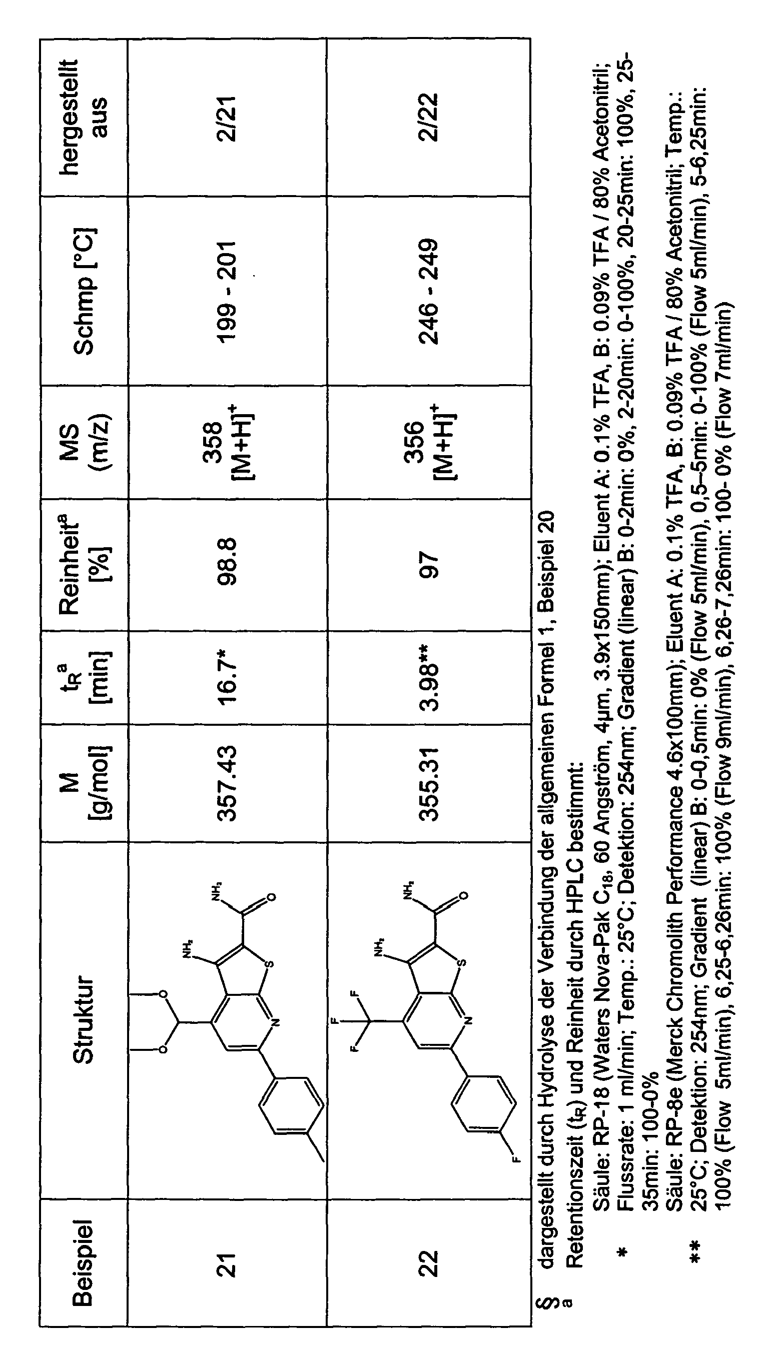

Exemplarische Beispiele substituierter 2-Mercapto-nicotinonitrile der allgemeinen Formel 2 (s.Tab.2), die analog oder in Anlehnung an bekannte Darstellungsmethoden synthetisiert wurden.

**Tab.2: Verbindungen der allgemeinen Formel 2**

| Verbindung | R₁ | R₂ | M [g/mol] | t_{R}^{a} [min] | Reinheit^{a} [%] |
|---|---|---|---|---|---|
| 2/1 | 4-Methyl-phenyl | CHF₂ | 276.3 | 4.00** | 94.6 |
| 2/2 | 2-Thienyl | CF₃ | 362.4 | 13.9* | 87 |
| 2/3 | 2-Furanyl | CF₃ | 346.3 | 13.2* | 89.6 |
| 2/4 | 1-Naphthyl | CF₃ | 330.3 | n.b. | n.b. |
| 2/5 | 2-Naphthyl | CF₃ | 330.3 | 3.94** | 80.6 |
| 2/6 | Phenyl | CF₃ | 280.3 | 13.8* | 94.6 |
| 2/7 | 4-Methyl-phenyl | CF₃ | 294.3 | 3.73** | 94.1 |
| 2/8 | 4-Ethyl-phenyl | CF₃ | 308.3 | 3.95** | 73.8 |
| 2/9 | 4-*n*-Propyl-phenyl | CF₃ | 322.3 | 17.5* | 100 |
| 2/10 | 4-Isopropyl-phenyl | CF₃ | 322.3 | 16.2* | 77.0 |
| 2/11 | 4-tert.-Butyl-phenyl | CF₃ | 336.4 | 4.26** | 76.5 |
| 2/12 | 4-Methoxy-phenyl | CF₃ | 310.3 | 14.2* | 98.4 |
| 2/13 | 4-Ethoxy-phenyl | CF₃ | 324.3 | 16.1 * | 80.4 |
| 2/14 | 4-Chlor-phenyl | CF₃ | 314.7 | 4.17** | 81.7 |
| 2/15 | 4-Brom-phenyl | CF₃ | 359.2 | 3.74** | 63.4 |
| 2/16 | 3,4-Dimethyl-phenyl | CF₃ | 308.3 | 3.93** | 89.1 |
| 2/17 | 3,5-Dimethyl-phenyl | CF₃ | 308.3 | 3.99** | 81.9 |
| 2/18 | 3,4-Methylendioxy | CF₃ | 400.4 | 14.6* | 92.7 |
| 2/20 | 4-Methyl-phenyl | COOEt | 298.4 | 15.0* | 51.0 |
| 2/21 | 4-Methyl-phenyl | CH(OMe)₂ | 300.4 | 22.3* | 73.8 |
| 2/22 | 4-Fluor-phenyl | CF₃ | 298.3 | 2.89** | 85.3 |

| | | | | | |
|---|---|---|---|---|---|
| ^{a} Retentionszeit (t_{R}) und Reinheit durch HPLC bestimmt: | | | | | |
| * Säule: RP-18 (Waters Nova-Pak C₁₈, 60 Angström, 4µm, 3.9x150mm); Eluent A: 0.1% TFA, B: 0.09% TFA / 80% Acetonitril; Flussrate: 1 ml/min; Temp.: 25°C; Detektion: 254nm; Gradient (linear) B: 0-2min: 0%, 2-20min: 0-100%, 20-25min: 100%, 25-35min: 100-0% | | | | | |
| ** Säule: RP-8e (Merck Chromolith Performance 4.6x100mm); Eluent A: 0.1 % TFA, B: 0.09% TFA / 80% Acetonitril; Temp.: 25°C; Detektion: 254nm; Gradient (linear) B: 0-0,5min: 0% (Flow 5ml/min), 0,5-5min: 0-100% (Flow 5ml/min), 5-6,25min: 100% (Flow 5ml/min), 6,25-6,26min: 100% (Flow 9ml/min), 6,26-7,26min: 100- 0% (Flow 7ml/min) | | | | | |

## Patentansprüche

1. 3-Amino-6-aryl(bzw. heteroaryl)-4-R²-thieno[2,3-b]pyridin-2-carbonsäureamide der allgemeinen Formel 1, 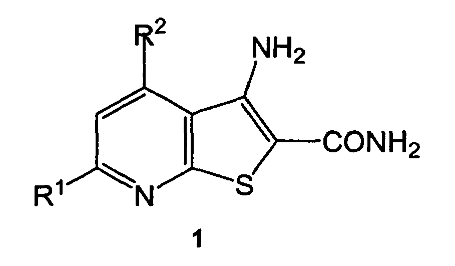 worin bedeuten:
R¹
- Phenyl, 4-R³-Phenyl, 3-R³-Phenyl, 2-R³-Phenyl, 2-R³,3-R³-Phenyl, 2-R³,4-R³-Phenyl, 2-R³,5-R³-Phenyl, 2-R³,6-R³-Phenyl, 3-R³,4-R³-Phenyl, 3-R³,4-R³,5-R³-Phenyl,
- 1-Naphthyl, 2-Naphthyl (jeweils ggf. mit R³ substituiert),
- 1,2,3,4-Tetrahydronaphth-5-yl, 1,2,3,4-Tetrahydronaphth-6-yl (jeweils ggf. mit R³ substituiert),
- 1-Anthryl, 2-Anthryl (jeweils ggf. mit R³ substituiert),
- mono- oder bicyclische gesättigte oder ein- oder mehrfach ungesättigte Heterocyclen mit 5 -14 Ringatomen, darunter 1-4 Heteroatome, die vorzugsweise N, O, S und Se sind (jeweils ggf. mit R³ substituiert),
R²
- CH₂F, CHF₂, CF₃, CH₂Br, CHBr₂, CBr₃, CH₂Cl, CHCl₂, CCl₃, CHFCH₃, CF₂CH₃,
- CH₂OAlk, CH₂SAlk, CH₂S(O)Alk, CH₂S(O)₂Alk ("Alk" jeweils C₁₋₃),
- CH(OAlk)₂, CH(SAlk)₂ ("Alk" jeweils C₁₋₃);
R³
- C₁₋₄Alkyl, C₁₋₄Alkoxy, Benzyl, Benzyloxy (jeweils ggf. mit R⁴ substituiert),
- Trifluormethoxy, Difluormethoxy, Fluormethoxy,
- C₃₋₇Cycloalkyl, C₃₋₇Cycloalkenyl, C₃₋₇Cycloalkoxy,
- Alkylthio, Alkylsulfinyl und Alkylsulfonyl (jeweils C₁₋₄),
- Phenyl, 4-R⁴-Phenyl, 3-R⁴-Phenyl, 2-R⁴-Phenyl, 2-R⁴,3-R⁴-Phenyl, 2-R⁴,4-R⁴-Phenyl, 2-R⁴,5-R⁴-Phenyl, 2-R⁴,6-R⁴-Phenyl, 3-R⁴,4-R⁴-Phenyl, 3-R⁴,4-R⁴,5-R⁴-Phenyl,
- Phenoxy, 4-R⁴-Phenoxy, 3-R⁴-Phenoxy, 2-R⁴-Phenoxy, 2-R⁴,3-R⁴-Phenoxy, 2-R⁴,4-R⁴-Phenoxy, 2-R⁴,5-R⁴-Phenoxy, 2-R⁴,6-R⁴-Phenoxy, 3-R⁴,4-R⁴-Phenoxy, 3-R⁴,4-R⁴,5-R⁴-Phenoxy,
- Pyrid-2-yl, Pyrid-3-yl, Pyrid-4-yl, Thien-2-yl, Fur-2-yl, Imidazol-2-yl, Oxazol-2-yl, Isoxazol-2-yl, Pyrrazol-4-yl,
- C₁₋₇Alkacyl (ggf. mit R⁴ substituiert),
- Formyl, Benzoyl, 1- und 2-Naphthoyl (jeweils ggf. mit R⁴ substituiert),
- Fluor, Chlor, Brom, Iod, Nitro, Hydroxy, Sulfhydryl, Cyano, Rhodano,
- Carboxyl, C₁₋₄Alkoxycarbonyl;
- CONH₂, CONHAlk und CONAlk₂ (mit "Alk" jeweils C₁₋₆),
- SO₃H, SO₂OAlk, SO₂NHAlk, SO₂NAlk₂ (mit "Alk": C₁₋₅),
- Chlor, Brom, Iod, Fluor,
- Amino, C₁₋₆Alkylamino, Di(C₁₋₅)alkylamino,
- Morpholino, Thiomorpholino, Thiomorpholino-S,S-Dioxid, Pyrrolidin-1-yl,
- Piperidin-1-yl, Piperazin-1-yl, 4-Methyl-piperazin-1-yl, 4-Hydroxyethyl-1-piperazin-1-yl, 4-Phenyl-piperazin-1-yl,
- Cycloalkylamino, C₃₋₁₄Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo-bzw. heterocyclischen Ringen mit R⁴ substituiert);
R³,4-R³
- OCH₂O, OCH₂CH₂O, SCH₂S, SCH₂CH₂S;
R⁴
- C₁₋₄Alkyl, C₁₋₄Alkoxy,
- Trifluormethoxy, Difluormethoxy, Fluormethoxy,
- Alkylthio, Alkylsulfinyl und Alkylsulfonyl (jeweils C₁₋₄),
- Fluor, Chlor, Brom, lod, Nitro, Hydroxy, Sulfhydryl, Cyano, Rhodano,
- Carboxyl, C₁₋₄Alkoxycarbonyl;
- CONH₂, CONHAlk und CONAlk₂ (mit "Alk" jeweils C₁₋₆),
- SO₃H, SO₂OAlk, SO₂NHAlk, SO₂NAlk₂ (mit "Alk": C₁₋₅),
- Chlor, Brom, lod, Fluor,
- Amino, C₁₋₆Alkylamino, Di(C₁₋₅)alkylamino,
sowie deren Solvate, einschließlich der pharmazeutisch akzeptablen Salze und Prodrug-Formulierungen und deren aktive Metabolite und gegebenenfalls deren Tautomere,
wobei die folgende Verbindung der Formel 1 ausgenommen ist:
R¹: Thien-2-yl; R²: CF₃.

2. Verbindung gemäß Anspruch 1, wobei diese ausgewählt ist aus:
3-Amino-6-phenyl-4-trifluormethyl-thieno[2,3-b]pyridin-2-carbonsäureamid;
3-Amino-4-difluormethyl-6-(4-methyl-phenyl)-thieno[2,3-b]pyridin-2-carbonsäureamid;
3-Amino-6-(4-methyl-phenyl)-4-trifluormethyl-thieno[2,3-b]pyridin-2-carbonsäureamid;
3-Amino-6-(4-methoxy-phenyl)-4-trifluormethyl-thieno[2,3-b]pyridin -2-carbonsäureamid;
3-Amino-6-(4-ethyl-phenyl)-4-trifluormethyl-thieno[2,3-b]pyridin-2-carbonsäureamid;
3-Amino-6-(4-ethoxy-phenyl)-4-trifluormethyl-thieno[2,3-b]pyridin -2-carbonsäureamid;
3-Amino-6-(4-isopropyl-phenyl)-4-trifluormethyl-thieno[2,3-b]pyridin -2-carbonsäureamid;
3-Amino-6-(4-propyl-phenyl)-4-trifluormethyl-thieno[2,3-b]pyridin-2-carbonsäureamid;
3-Amino-6-(4-methylthio-phenyl)-4-trifluormethyl-thieno[2,3-b]pyridin-2-carbonsäureamid
3-Amino-6-(3,4-dioxomethylen-phenyl)-4-trifluormethyl-thieno[2,3-b]pyridin -2-carbonsäureamid;
3-Amino-6-(3,4-dioxoethylen-phenyl)-4-trifluormethyl-thieno[2,3-b]pyridin -2-carbonsäureamid;
3-Amino-6-(3,4-dimethyl-phenyl)-4-trifluormethyl-thieno[2,3-b]pyridin -2-carbonsäureamid;
3-Amino-6-(2-naphthyl)-4-trifluormethyl-thieno[2,3-b]pyridin-2-carbonsäureamid;
3-Amino-6-(2-thienyl)-4-trifluormethyl-thieno[2,3-b]pyridin-2-carbonsäureamid;
3-Amino-6-(4-chlor-phenyl)-4-trifluormethyl-thieno[2,3-b]pyridin-2-carbonsäureamid;
3-Amino-6-(4-brom-phenyl)-4-trifluormethyl-thieno[2,3-b]pyridin-2-carbonsäureamid;
3-Amino-6-(4-fluor-phenyl)-4-trifluormethyl-thieno[2,3-b]pyridin-2-carbonsäureamid;
3-Amino-6-(4-*tert*.butyl-phenyl)-4-trifluormethyl-thieno[2,3-b]pyridin -2-carbonsäureamid;
3-Amino-6-(4-methylsulfinyl-phenyl)-4-trifluormethyl-thieno[2,3-b]pyridin -2-carbonsäureamid;
3-Amino-6-(2-furyl)-4-trifluormethyl-thieno[2,3-b]pyridin
-2-carbonsäureamid; 3-Amino-4-methoxymethyl-6-(4-methyl-phenyl)thieno[2,3-b]pyridin
-2-carbonsäureamid;

3. Pharmazeutische Zusammensetzung enthaltend mindestens eine der nachfolgenden Verbindungen der Formel 1 sowie ggf. pharmazeutisch verträgliche Hilfsstoffe und/oder Träger: 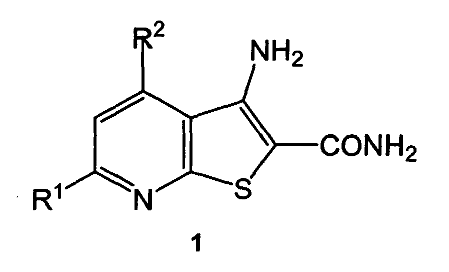 worin bedeuten:
R¹
- Phenyl, 4-R³-Phenyl, 3-R³-Phenyl, 2-R³-Phenyl, 2-R³,3-R³-Phenyl, 2-R³,4-R³-Phenyl, 2-R³,5-R³-Phenyl, 2-R³,6-R³-Phenyl, 3-R³,4-R³-Phenyl, 3-R³,4-R³,5-R³-Phenyl,
- 1-Naphthyl, 2-Naphthyl (jeweils ggf. mit R³ substituiert),
- 1,2,3,4-Tetrahydronaphth-5-yl, 1,2,3,4-Tetrahydronaphth-6-yl (jeweils ggf. mit R³ substituiert),
- 1-Anthryl, 2-Anthryl (jeweils ggf. mit R³ substituiert),
- mono- oder bicyclische gesättigte oder ein- oder mehrfach ungesättigte Heterocyclen mit 5 -14 Ringatomen, darunter 1-4 Heteroatome, die vorzugsweise N, O, S und Se sind (jeweils ggf. mit R³ substituiert),
R²
- CH₂F, CHF₂, CF₃, CH₂Br, CHBr₂, CBr₃, CH₂Cl, CHCl₂, CCl₃, CHFCH₃, CF₂CH₃,
- CH₂OAlk, CH₂SAlk, CH₂S(O)Alk, CH₂S(O)₂Alk ("Alk" jeweils C₁₋₃),
- CH(OAlk)₂, CH(SAlk)₂ ("Alk" jeweils C₁₋₃);
R³
- C₁₋₄Alkyl, C₁₋₄Alkoxy, Benzyl, Benzyloxy (jeweils ggf. mit R⁴ substituiert),
- Trifluormethoxy, Difluormethoxy, Fluormethoxy,
- C₃₋₇Cycloalkyl, C₃₋₇Cycloalkenyl, C₃₋₇Cycloalkoxy,
- Alkylthio, Alkylsulfinyl und Alkylsulfonyl (jeweils C₁₋₄), - Phenyl, 4-R⁴-Phenyl, 3-R⁴-Phenyl, 2-R⁴-Phenyl, 2-R⁴,3-R⁴-Phenyl, 2-R⁴,4-R⁴-Phenyl, 2-R⁴,5-R⁴-Phenyl, 2-R⁴,6-R⁴-Phenyl, 3-R⁴,4-R⁴-Phenyl, 3-R⁴,4-R⁴,5-R⁴-Phenyl,
- Phenoxy, 4-R⁴-Phenoxy, 3-R⁴-Phenoxy, 2-R⁴-Phenoxy, 2-R⁴,3-R⁴-Phenoxy, 2-R⁴,4-R⁴-Phenoxy, 2-R⁴,5-R⁴-Phenoxy, 2-R⁴,6-R⁴-Phenoxy, 3-R⁴,4-R⁴-Phenoxy, 3-R⁴,4-R⁴,5-R⁴-Phenoxy,
- Pyrid-2-yl, Pyrid-3-yl, Pyrid-4-yl, Thien-2-yl, Fur-2-yl, Imidazol-2-yl, Oxazol-2-yl, Isoxazol-2-yl, Pyrrazol-4-yl,
- C₁₋₇Alkacyl (ggf. mit R⁴ substituiert),
- Formyl, Benzoyl, 1- und 2-Naphthoyl (jeweils ggf. mit R⁴ substituiert),
- Fluor, Chlor, Brom, lod, Nitro, Hydroxy, Sulfhydryl, Cyano, Rhodano,
- Carboxyl, C₁₋₄Alkoxycarbonyl;
- CONH₂, CONHAlk und CONAlk₂ (mit "Alk" jeweils C₁₋₆),
- SO₃H, SO₂OAlk, SO₂NHAlk, SO₂NAlk₂ (mit "Alk": C₁₋₅),
- Chlor, Brom, Iod, Fluor,
- Amino, C₁₋₆Alkylamino, Di(C₁₋₅)alkylamino,
- Morpholino, Thiomorpholino, Thiomorpholino-S,S-Dioxid, Pyrrolidin-1-yl,
- Piperidin-1-yl, Piperazin-1-yl, 4-Methyl-piperazin-1-yl, 4-Hydroxyethyl-1-piperazin-1-yl, 4-Phenyl-piperazin-1-yl,
- Cycloalkylamino, C₃₋₁₄Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo-bzw. heterocyclischen Ringen mit R⁴ substituiert);
R³,4-R³
- OCH₂O, OCH₂CH₂O;
R⁴
- C₁₋₄Alkyl, C₁₋₄Alkoxy,
- Trifluormethoxy, Difluormethoxy, Fluormethoxy,
- Alkylthio, Alkylsulfinyl und Alkylsulfonyl (jeweils C₁₋₄),
- Fluor, Chlor, Brom, lod, Nitro, Hydroxy, Sulfhydryl, Cyano, Rhodano,
- Carboxyl, C₁₋₄Alkoxycarbonyl;
- CONH₂, CONHAlk und CONAlk₂ (mit "Alk" jeweils C₁₋₆),
- SO₃H, SO₂OAlk, SO₂NHAlk, SO₂NAlk₂ (mit "Alk": C₁₋₅),
- Chlor, Brom, lod, Fluor,
- Amino, C₁₋₆Alkylamino, Di(C₁₋₅)alkylamino,
sowie deren Solvate, einschließlich der pharmazeutisch akzeptablen Salze und Prodrug-Formulierungen und deren aktive Metabolite und gegebenenfalls deren Tautomere,
wobei die folgenden Verbindung der Formel 1 ausgenommen ist:
R¹: Thien-2-yl; R²: CF₃.

4. Pharmazeutische Zusammensetzung nach Anspruch 3 enthaltend zusätzlich einen oder mehrere der folgenden Wirkstoffe:
- Zytokin-Antagonisten
- Chemokin-Antagonisten
- Zytokin-Agonisten
- immunmodulatorische Wirkstoffe
- Substanz P-Antagonisten
- Bradykinin-Antagonisten
- PAF-Antagonisten
- Adenosin-Rezeptor-Antagonisten
- Antibiotika/Virostatika
- α-Mimetika
- Zytostatika
- β₂-Adrenoceptor Agonisten
- Leukotrien-Antagonisten (entweder Enzym-Inhibitoren [wie 5-Lipoxygenaseinhibitoren oder Arachidonsäure-Enzyminhibitoren] oder Rezeptorantagonisten),
- Antihistaminika (bevorzugt solche mit Mastzellen-stabilisierenden Eigenschaften oder Leukotrien-antagonisierenden Aspekten)
- Theophyllin
- Muscarinrezeptor-Antagonisten

5. Verwendung von mindestens eine der nachfolgenden Verbindungen der Formel 1 sowie ggf. pharmazeutisch verträgliche Hilfsstoffe und/oder Träger 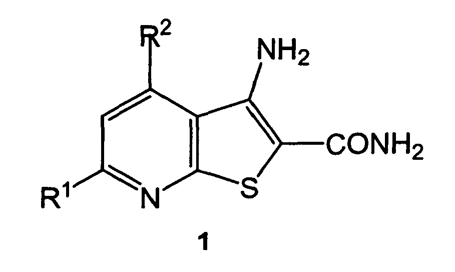 worin bedeuten:
R¹
- Phenyl, 4-R³-Phenyl, 3-R³-Phenyl, 2-R³-Phenyl, 2-R³,3-R³-Phenyl, 2-R³,4-R³-Phenyl, 2-R³,5-R³-Phenyl, 2-R³,6-R³-Phenyl, 3-R³,4-R³-Phenyl, 3-R³,4-R³,5-R³-Phenyl,
- 1-Naphthyl, 2-Naphthyl (jeweils ggf. mit R³ substituiert),
- 1,2,3,4-Tetrahydronaphth-5-yl, 1,2,3,4-Tetrahydronaphth-6-yl (jeweils ggf. mit R³ substituiert),
- 1-Anthryl, 2-Anthryl (jeweils ggf. mit R³ substituiert),
- mono- oder bicyclische gesättigte oder ein- oder mehrfach ungesättigte Heterocyclen mit 5 -14 Ringatomen, darunter 1-4 Heteroatome, die vorzugsweise N, O, S und Se sind (jeweils ggf. mit R³ substituiert),
R²
- CH₂F, CHF₂, CF₃, CH₂Br, CHBr₂, CBr₃, CH₂Cl, CHCl₂, CCl₃, CHFCH₃, CF₂CH₃,
- CH₂OAlk, CH₂SAlk, CH₂S(O)Alk, CH₂S(O)₂Alk ("Alk" jeweils C₁₋₃),
- CH(OAlk)₂, CH(SAlk)₂ ("Alk" jeweils C₁₋₃);
R³
- C₁₋₄Alkyl, C₁₋₄Alkoxy, Benzyl, Benzyloxy (jeweils ggf. mit R⁴ substituiert),
- Trifluormethoxy, Difluormethoxy, Fluormethoxy,
- C₃₋₇Cycloalkyl, C₃₋₇Cycloalkenyl, C₃₋₇Cycloalkoxy,
- Alkylthio, Alkylsulfinyl und Alkylsulfonyl (jeweils C₁₋₄),
- Phenyl, 4-R⁴-Phenyl, 3-R⁴-Phenyl, 2-R⁴-Phenyl, 2-R⁴,3-R⁴-Phenyl, 2-R⁴,4-R⁴-Phenyl, 2-R⁴,5-R⁴-Phenyl, 2-R⁴,6-R⁴-Phenyl, 3-R⁴,4-R⁴-Phenyl, 3-R⁴,4-R⁴,5-R⁴-Phenyl,
- Phenoxy, 4-R⁴-Phenoxy, 3-R⁴-Phenoxy, 2-R⁴-Phenoxy, 2-R⁴,3-R⁴-Phenoxy, 2-R⁴,4-R⁴-Phenoxy, 2-R⁴,5-R⁴-Phenoxy, 2-R⁴,6-R⁴-Phenoxy, 3-R⁴,4-R⁴-Phenoxy, 3-R⁴,4-R⁴,5-R⁴-Phenoxy,
- Pyrid-2-yl, Pyrid-3-yl, Pyrid-4-yl, Thien-2-yl, Fur-2-yl, Imidazol-2-yl, Oxazol-2-yl, Isoxazol-2-yl, Pyrrazol-4-yl,
- C₁₋₇Alkacyl (ggf. mit R⁴ substituiert),
- Formyl, Benzoyl, 1- und 2-Naphthoyl (jeweils ggf. mit R⁴ substituiert),
- Fluor, Chlor, Brom, Iod, Nitro, Hydroxy, Sulfhydryl, Cyano, Rhodano,
- Carboxyl, C₁₋₄Alkoxycarbonyl;
- CONH₂, CONHAlk und CONAlk₂ (mit "Alk" jeweils C₁₋₆),
- SO₃H SO₂OAlk, SO₂NHAlk, SO₂NAlk₂ (mit "Alk": C₁₋₅),
- Chlor, Brom, Iod, Fluor,
- Amino, C₁₋₆Alkylamino, Di(C₁₋₅)alkylamino,
- Morpholino, Thiomorpholino, Thiomorpholino-S,S-Dioxid, Pyrrolidin-1-yl,
- Piperidin-1-yl, Piperazin-1-yl, 4-Methyl-piperazin-1-yl, 4-Hydroxyethyl-1-piperazin-1-yl, 4-Phenyl-piperazin-1-yl,
- Cycloalkylamino, C₃₋₁₄Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo-bzw. heterocyclischen Ringen mit R⁴ substituiert);
R³,4-R³
- OCH₂O, OCH₂CH₂O;
R⁴
- C₁₋₄Alkyl, C₁₋₄Alkoxy,
- Trifluormethoxy, Difluormethoxy, Fluormethoxy,
- Alkylthio, Alkylsulfinyl und Alkylsulfonyl (jeweils C₁₋₄),
- Fluor, Chlor, Brom, Iod, Nitro, Hydroxy, Sulfhydryl, Cyano, Rhodano,
- Carboxyl, C₁₋₄Alkoxycarbonyl;
- CONH₂, CONHAlk und CONAlk₂ (mit "Alk" jeweils C₁₋₆),
- SO₃H, SO₂OAlk, SO₂NHAlk, SO₂NAlk₂ (mit "Alk": C₁₋₅),
- Chlor, Brom, lod, Fluor,
- Amino, C₁₋₆Alkylamino, Di(C₁₋₅)alkylamino,
sowie deren Solvate, einschließlich der pharmazeutisch akzeptablen Salze und Prodrug-Formulierungen und deren aktive Metabolite und gegebenenfalls deren Tautomere
zur Herstellung eines Arzneimittels zur Inhibierung von TNF-alpha.

6. Verwendung nach Anspruch 5 zur Behandlung von Rheumatoider Arthritis, Osteoarthritis, Osteoporosis, Asthma bronchiale, chronischer obstruktiver pulmonäre Erkrankung (COPD), Autoimmun-Erkrankungen wie z.B. Systemischer Lupus Erytematodes (SLE), Sklerodermie, Crohns Disease, Colitis ulcerosa, Multipler Sklerose, Guillain-Barré-Syndrom, Crohn's Disease, Colitis ulcerosa, Psoriasis, Graft-versus-Host-Disease, systemischem Alzheimer-Erkrankung, insulin-abhängiger Diabetes mellitus, Respiratorischem Distress-Syndrom beim Erwachsenen (ARDS), Sepsis, cerebrale Form der Malaria, Guillain-Barre-Syndrom, Graft-versus-Host-Disease (GvHD), multiples Organversagen nach Trauma, aktuter Glomerulonephritis, akute und chronische Schmerzen, Arteriosklerose, Herzinfarkt, Schlaganfall,, entzündlichen Dermatosen wie Psoriasis, atopische Dermatitis, Alopecie, akuter Meningitis, Vasculitis, Uveitis" atopischer Dermatitis, cardiovaskuläre Erkrankungen, virale Erkrankungen und hier insbesondere retrovirale Erkrankungen wie z.B. das erworbene Immundefizienz Syndrom (AIDS) sowie Krebs, insbesondere Entartungen des blutbildenden Systems, neurodegenarativen Erkrankungen wie z.B. Mb. Alzheimer, Mb. Parkinson, Rhinitis allergica, allergische Konjunktivitis, Myastenia Gravis, und Sarkoidose.
